# EUROPEAN PATENT APPLICATION

(11) **EP 4 397 674 A1**
(43) Date of publication of application: **10.07.2024**
(21) Application number: 23382003.4
(22) Date of filing: 03.01.2023
(51) Int. Cl.: C07K 7/06, A61K 38/08, A61P 3/00, A61P 25/00

(54) **CASPASE-2 INHIBITOR COMPOUNDS**

(71) Applicant: Kintsugi Therapeutics S.L., 08018 Barcelona (ES)
(72) Inventor: HERRERO MOLINA, Maria del Carmen, 08018 Barcelona (ES); CUSACHS FARRARÓ, Marc, 08018 Barcelona (ES); DRAG, Marcin, 50-370 Wroclaw (PL); POREBA, Marcin, 50-370 Wroclaw (PL); ÁVILA ZARAGOZÁ, Matías, 31008 Pamplona (ES); GARCÍA FERNÁNDEZ-BARRENA, Maite, 31008 Pamplona (ES)
(74) Representative: Hoffmann Eitle

(57) **Abstract**

The invention relates to compounds of formula (II): 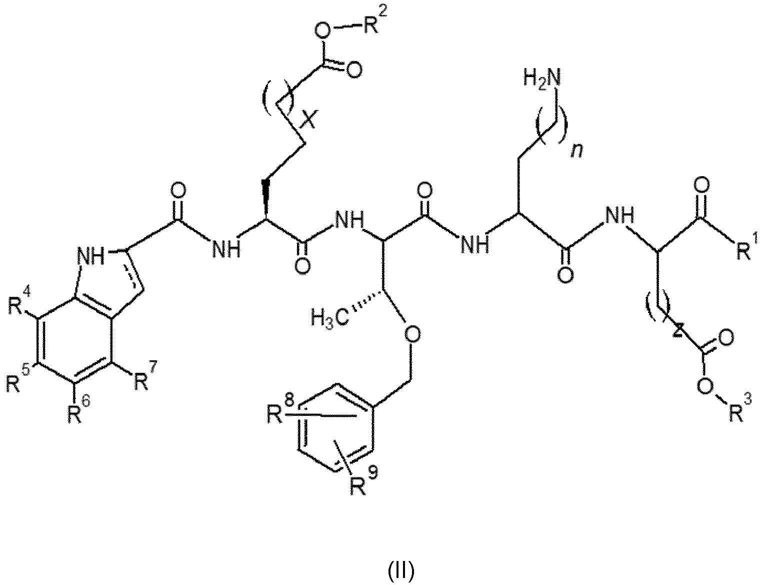 or pharmaceutically acceptable salts, solvates or stereoisomers thereof, to pharmaceutical compositions comprising them and to their use in the treatment and/or prevention of diseases or disorders mediated by Caspase-2.

## Description

### FIELD OF THE INVENTION

The present invention relates to new peptide derivatives, pharmaceutical compositions comprising them and their use in the prevention and/or treatment of Caspase-2 mediated diseases or disorders.

### STATE OF THE ART

Caspases are a family of evolutionary conserved cysteine-dependent endoproteases that hydrolyze their substrates after specific aspartic acid residues (Lamkanfi, 2002). Caspases have been associated with a wide range of biological activities such as apoptosis (Ramirez and Salvesen, 2018), inflammation (Vande Walle, 2016), cell differentiation (Fernando, 2002) and metabolism (Shalini, 2015).

Caspases are classified in two major groups: those involved in the regulation of inflammatory processes (-1, -4, -5, -11, -12) and those that are central to the initiation and execution of apoptosis (2, -3, -7, -8, -9, -10) (Shalini, 2015). Apoptotic caspase proenzymes are classified as either initiator or executioner caspases based on their role in the apoptotic program. Initiator caspases (e.g., caspases-2, -8, and -9), which are important in upstream signalling, become activated through the binding of adaptor proteins to their pro-domains. This binding facilitates their oligomerization into high molecular weight protein complexes, which triggers their dimerization and processing into active enzymes. Once active, initiator caspases activate executioner caspases (e.g., caspases-3 and -7) via proteolytic cleavage. Executioner caspases then go on to cleave protein substrates, leading to an organized dismantling of the cell and ultimately cell death.

Initiator caspase activation during apoptosis is mediated by two main pathways: the mitochondrial or intrinsic pathway and the death receptor or extrinsic pathway. The intrinsic pathway is activated in response to cellular stress (e.g., ROS, cytotoxic drugs, DNA damage) and results in the activation of the initiator caspase-2 (Shalini, 2015). Active caspase-2 cleaves Bid and truncated Bid (tBid) activate mitochondrial permeabilization through regulation of Bax and Bak (Enoksson, 2004). Mitochondrial cytochrome c is then released to the cytosol inducing the oligomerization of Apaf-1, forming a large heptameric complex known as the apoptosome (Bao, 2007). The apoptosome recruits and activates the initiator caspase, caspase-9, which can directly cleave and activate the executioner caspases, caspase-3 and -7(Bao, 2007).

The extrinsic apoptotic pathway is initiated at the plasma membrane by the binding of a ligand to its extracellular death receptor (e.g., TNFR, Fas, TRAIL) (Gaur, 2003). This mediates recruitment and activation of caspase-8 or -10, through the death-inducing signaling complex (DISC) comprising FAS-associated death domain protein (FADD) and/or TNFR-associated death domain protein (TRADD) and other components. Caspase-8 also cleaves BID to a truncated form (tBID), which engages the mitochondrial pathway to amplify the apoptotic response (Li, 1998). Once initiator caspases are activated through the extrinsic or intrinsic apoptosis pathways, they mediate activation of effector caspases-3, -6 and -7 (Bao, 2007).

Caspase-2, originally named Need-2 (in mice) or Ich-1 (in humans), is the most conserved caspase across species, sharing a 55% similarity with C.elegans (Yuan, 1993; Wang, 1994;Kumar, 1994). Caspase-2 contains an N-terminal caspase recruitment domain (CARD), followed by a large subunit containing the active site (p19) and a small subunit (p12). Thus, caspase-2 is most like caspase-9, the initiator of the intrinsic pathway of apoptosis (Li and Yuan, 2008). However, in contrast to conventional initiator caspases, such as caspase-9 or the apical caspase of extrinsic apoptosis, caspase-8, caspase-2 does not process apoptosis effectors that need to be cleaved by initiators for their activation, such as caspase-3, -6 or -7 (Guo et al., 2002; Van de Craen et al., 1999). In contrast to other initiator caspases, caspase-2 undergoes autocatalytic cleavage after dimerization and does not require cleavage for its initial activation (B.C. Baliga 2004).

Caspase-2 possesses several unique characteristics, including the presence of a nuclear localization signal, which plays a central role in the triggering of apoptotic pathways after DNA damage in some cellular models. In addition, caspase-2 has been reported to be non-apoptotic signaling pathways, including de novo lipogenesis (Kim, 2018), metabolism regulation (Nutt, 2005), tumour suppression (Kumar, 1995), mitotic catastrophe (Vitale, 2011) cell cycle regulation (Sidi, 2008), and DNA repair (Vigneswara, 2020).

The loss of vital cells within healthy tissues due to an increased apoptosis contributes to the development and progression of many human disorders as well as environmental, medical toxicities and pathogens (Singh, 2019).

Increased caspase-2 induced apoptosis has been described in different diseases or pathological situations.
- Retinal ganglion cell (RGC) loss after optic nerve damage is a hallmark of certain human ophthalmic diseases as ischemic optic neuropathy. In a rat model of optic nerve transection caspase-2 was found to be expressed and cleaved in RGC (Ahmed, 2011).
- In neonatal ischemic brain damage, ischemia injury triggers multiple pathways of oxidant stress, inflammation, and excitotoxicity that lead to massive cellular death in the ischemic area by apoptosis mediated by caspase 2-activation (Carlsson, 2011; Chauvier, 2011).
- In stroke apoptosis of cerebral neurons is the main pathological change occurring in the peri-infarct zone after transient global ischemia, causing ischemia/reperfusion (I/R) damage. Cerebral neuronal apoptosis can cause hemiplegia, death, or cognitive impairment after a stroke (Turkmen, 2011). In an animal model of brain stroke, caspase 2 expression and activation were increased after I/R.
- In Alzheimer's disease (AD) and other tauopathies, the tau protein forms fibrils, which are believed to be neurotoxic. Caspase-2 cleaves tau at Asp314 and this results in impairment of cognitive and synaptic function in animal and cellular models. The truncation product, Δtau314, resists fibrillation and is present at higher levels in brains from cognitively impaired mice and humans with AD. The expression of tau mutants that resisted caspase-2 cleavage prevented tau from infiltrating spines, dislocating glutamate receptors and impairing synaptic function in cultured neurons, and it prevented memory deficits and neurodegeneration in mice (Zhao, 2016).
- Caspase-2 promotes obesity, metabolic syndrome, and non-alcoholic fatty liver disease (Machado, 2016; Kim 2018). Caspase-2 expression strongly correlated with liver disease severity in patients with NAFLD (Machado, 2015; Kim, 2018).

The strong evidence of increased apoptosis having a role in many diseases has prompted efforts to drug this pathway for therapeutic benefit. The first generation of caspase inhibitors were reversible aldehyde peptides that showed limited therapeutic benefit due to the lack of selectivity and the different warheads used.

In the first generations of inhibitors several peptide sequences were developed to supposedly inhibit the different caspases in a selective manner, for example the Ac-DEVD-CHO (a preferential inhibitor of Caspase-3 and Caspase-7) and Ac-VDVAD-CHO (a preferential inhibitor of Caspase-2, -3, and -7).

In order to effectively inhibit apoptosis, selectivity against caspase-3 is critically important because of the high concentration of this caspase relative to other caspases in almost all tissues and of its promiscuous nature (McStay, 2008). Moreover, inhibitors non-selective against caspase 2 have limited clinical benefit for direct apoptosis inhibition because they work downstream of the mitochondrial outer membrane pore and cannot reverse the profound damage to mitochondrial function that is induced by Caspase-2-mediated pore formation (Singh, 2019). Furthermore, selectivity can also be critical in situations where caspase-8 (extrinsic apoptosis) is also activated, and a selective caspase-2 (intrinsic apoptosis) inhibition is necessary.

The inhibitor Ac-VDVAD-CHO is more potent against caspase-3 than to caspase-2. This raises some doubts about the validity of the data generated with this reagent when employed in a cellular context as a 'selective' caspase-2 inhibitor. Several publications have already expressed the specificity concerns regarding existing inhibitors and stressed the urgent need for more selective inhibitors in the caspase field (Pereira, 2008; Berger, 2006; McStay, 2008; Benkova, 2009; Yun, 2007; Krumschnabel, 2009; Kitevska, 2009; Schweizer, 2007; Poreba, 2019 ("Caspase selective reagents for diagnosing apoptotic mchanisms" Poreba et al. Cell Death and Differentiation. In this paper (mainly in supplemental section) we provided a detailed kinetic analysis of caspase inhibitors/probes made based on natural amino acid sequences).

There are several types of warheads used in caspase inhibitors design; however, all of them work in a similar manner. Their mechanism of action relies on nucleophilic attack of the active site cysteine on the electrophilic center, thus forming a transitional (reversible) or covalent (irreversible) caspase-inhibitor complex (Evans, 2006). The most significant feature of a particular warhead is its ability to target only active site cysteine residue, omitting other free nucleophiles in the proteome. Thus, the catalytic mechanism of the targeted proteolytic enzyme plays an important role in the selection of an appropriate warhead.

It is important that the thiol group of the catalytic cysteine residue in cysteine proteases is more polarizable than the hydroxyl group on the catalytic serine or threonine, therefore electrophiles used as a warhead for cysteine proteases can be softer than ones for serine or threonine proteases (Powers, 2002). Thus, caspases are most extensively studied with inhibitors containing warheads such as diazomethylketons, epoxides and halo- and acyloxy-methylketons. The major advantages of these warheads are their ease of synthesis, good bioavailability, and active site Cys-selective reactivity. The most used and commercially available caspases inhibitors warheads are fluoro-(FMK or -CH2F), chloro- (CMK or -CH2CI), or acyloxy-methylketones (AOMK) (Poreba, 2015, ("Small molecule active site directed tools for studying human caspases" Poreba et al. Chemical Reviews, 2015)). The major advantages of these warheads are their ease of synthesis, good bioavailability, and active site Cys-selective reactivity (Sanman, 2014; Powers, 2002).

FMK-based inhibitors were the first and so far, they have dominated research in this field. One of the advantages of FMKs is that ketone reagents penetrate the plasma membrane and is relatively nontoxic to the cell (Van Noorden, 2001 ("The history of Z-VAD-FMK, a tool for understanding the significance of caspase inhibition" Van Noorden, Acta Histochemica, 2001)). The use of FMK inhibitors has also some disadvantages: cross-reactivity of such probes with other cysteine proteases such as legumains, cathepsin B and cathepsin H and production of high labeling background from nonspecific binding of reactive the FMK group (Rozman-Pungercar, 2003; Schotte, 1999). Inhibitors containing the FMK-warhead have been shown to be toxic in vivo because of the release of the fluoroacetate group particularly in the liver leading to the inhibition of aconitase. Thus, the development of inhibitors with a FMK group, was abandoned in the preclinical phase because of its hepatotoxicity (Citarella, 2020).

The AOMKs are the weakest electrophile among this group, thus they are most suitable for the development of caspase inhibitors because of little cross reactivity with other bionucleophiles. Therefore, the weaker electrophilicity allows them to react with caspases more specifically and display reduced cross-reactivity with other Cys-dependent proteases (Poreba et al., Chemical Reviews (2015) BI-BJ).

Down-modulation of caspase-2 expression has been effective preventing apoptosis in several models:
- In a model of retinal ganglion cell (RGC) loss after optic nerve damage, inhibition of caspase-2 expression by siRNA had a neuroprotective effect by significantly enhancing RGC survival over a period of at least 30 days (Ahmed, 2011).
- In neonatal ischemic brain damage, genetic (Carlsson, 2011) or pharmacological (Chauvier, 2011) inhibition of caspase 2 reduces cortical and white matter damage in the neonatal brain after excitotoxicity, arterial stroke, and hypoxia.
- In stroke apoptosis, treatment with a microRNA (miR-1247-3p) inhibited caspase-2 expression and attenuated neuronal apoptosis (Zhang, 2019).
- In animal models of Alzheimer's disease (AD) and other tauopathies, decreasing the levels of caspase-2 restored long-term memory in mice that had existing deficits (Zhao, 2016).
- Caspase-2 depletion protected mice with methionine/choline-deficient (MCD) diet-induced steatohepatitis from hepatocyte apoptosis and fibrosis progression (Machado, 2015).

Compounds able to inhibit Caspase-2 activity have been reported for example in WO 2005/105829 and EP2670774. However, these known Caspase-2 inhibitors also have a too high activity with respect to Caspase-3. They this cannot be qualified as selective Caspase-2 inhibitors. More recently, a series of reversible Caspase-2 inhibitors has been reported. When evaluated in vitro on human recombinant Caspases, these compounds were found to preferentially inhibit Caspase-2 but have moderate effects in cellular assays and structural properties that are incompatible with in vivo use (Maillard, 2011). WO2017/162674 and WO2019/068538 disclose peptide compounds containing five amino acid units as Caspase-2 inhibitors.

Poreba et al. (2019) also disclose Caspase-2 inhibitors constituted by five amino acid units in L-stereochemistry. Compound NH-23-C2 (NH-ldc-hGlu-Thr(Bzl)-Ser-Asp) was found to have the highest Caspase-2 inhibitory activity among the assayed compounds but still with no selectivity for Caspase-3 and limited selectivity for Caspase-8.

Accordingly, there is still a need for potent and/or selective Caspase-2 inhibitors more particularly with a significant reduced activity against Caspase-3/Caspase-8. In particular, it would be highly advantageous to provide more selective and efficient caspase-2 inhibitors for use in the prevention and/or treatment of diseases and/or injuries in which Caspase-2 activity is implicated such as nonalcoholic steatohepatitis (NASH) or nonalcoholic fatty liver disease (NAFLD), obesity, metabolic syndrome, cirrhosis, neonatal brain ischemia, heart ischemia and chronic degenerative diseases like for instance Alzheimer's disease.

It would also be very advantageous to provide more efficacious and selective Caspase-2 inhibitors for use as an activity-based probe to specifically detect Caspase-2 activity.

The compounds of the invention aim to meet these needs.

### BRIEF DESCRIPTION OF THE FIGURES

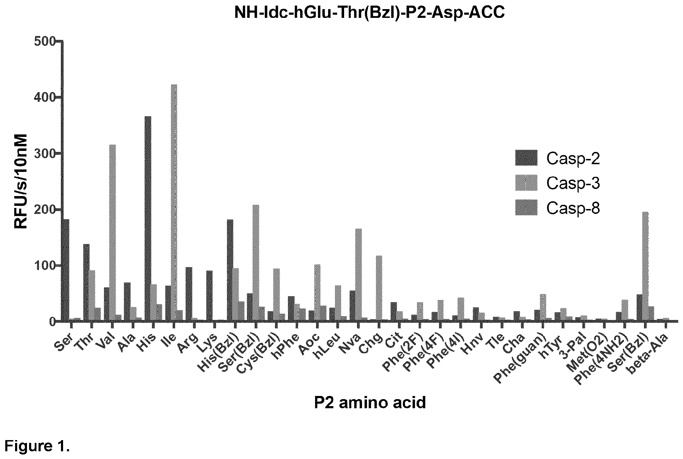

**Figure 1****.** P2 substrates screening towards caspase-2, caspase-3 and caspase-8. The x axis represents abbreviated names of *L*-amino acids at the P2 position in NH-ldc-hGlu-Thr(Bzl)-P2-Asp-ACC, whereas the y axis represents the substrate hydrolysis rate (RFU/s) per 10nM of a particular active or "active-site titrated" caspase. The concentration of active caspases were determined by active site titration.

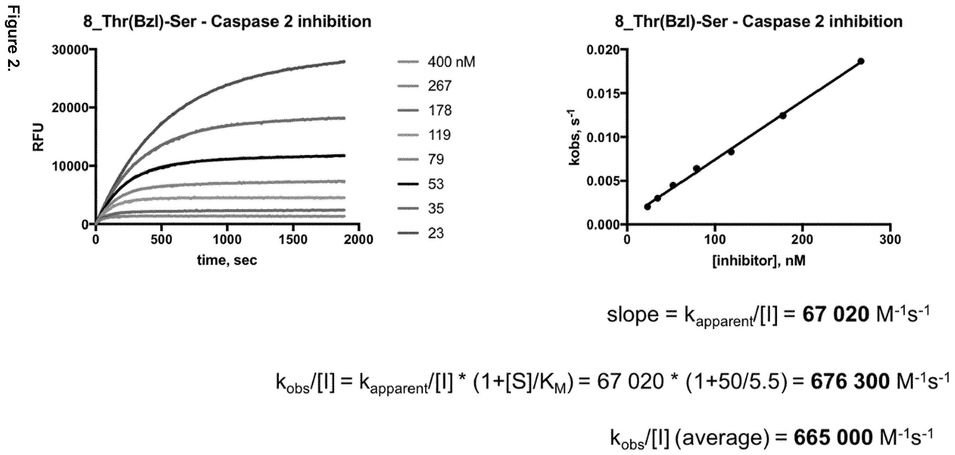

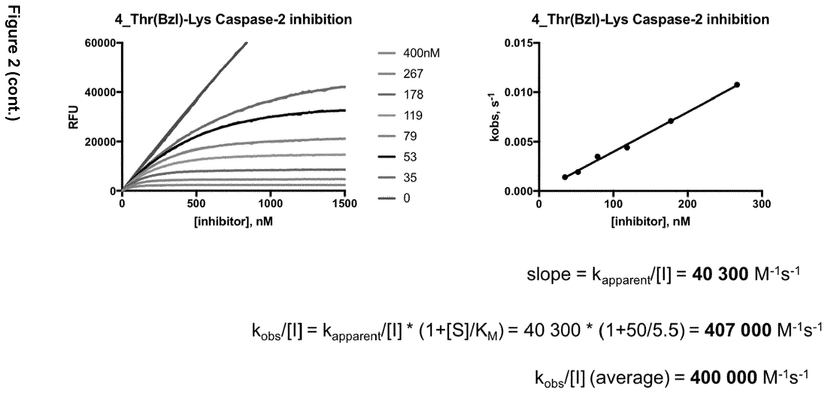

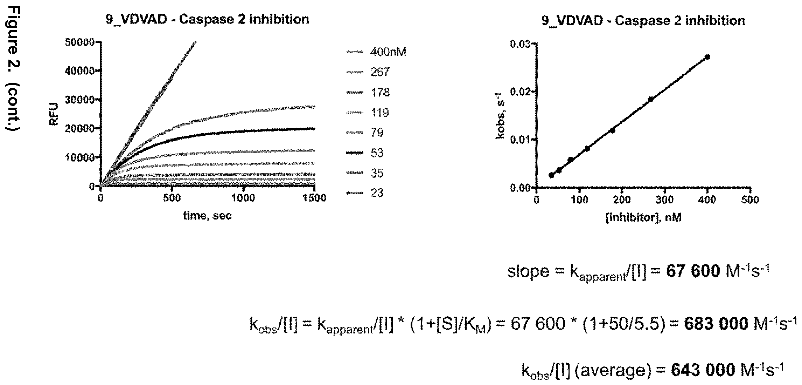

**Figure 2****.** Raw data for calculation of kobs/I inhibition parameter of NH-23-C2 synthesized inhibitors towards caspases-2. The kobs/I parameter was measured under pseudo-first-order kinetic conditions ([I] >> [E]). NH-ldc-hGlu-Thr(Bzl)-Ser-Asp-ACC was used as a substrate. The ACC fluorescence was monitored using 355 nm (excitation) and 460 nm (emission) wavelengths. Second-order rate of inhibition (kobs/I) was determined in a at least three independent experiments and presented as an average value. GraphPad Prism 7 software was used for calculations.

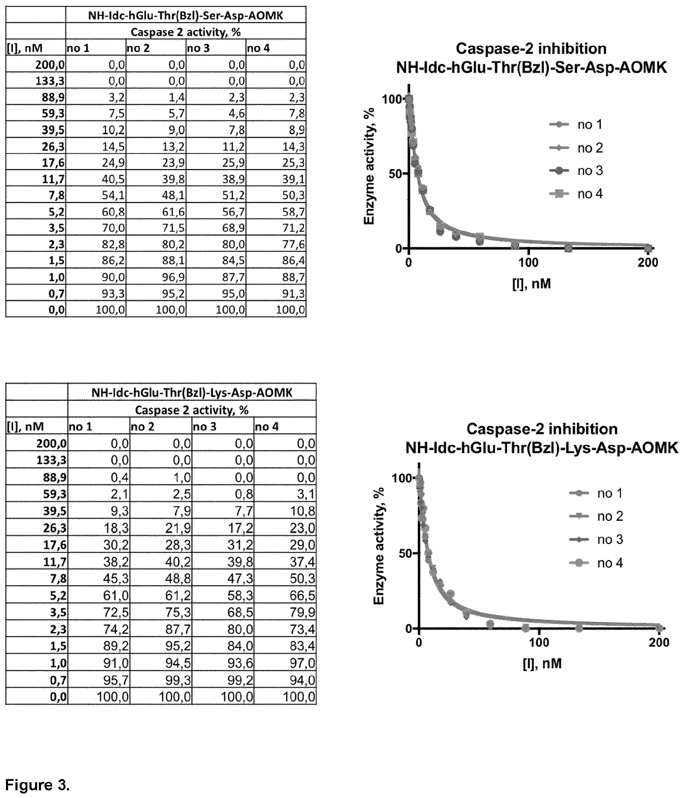

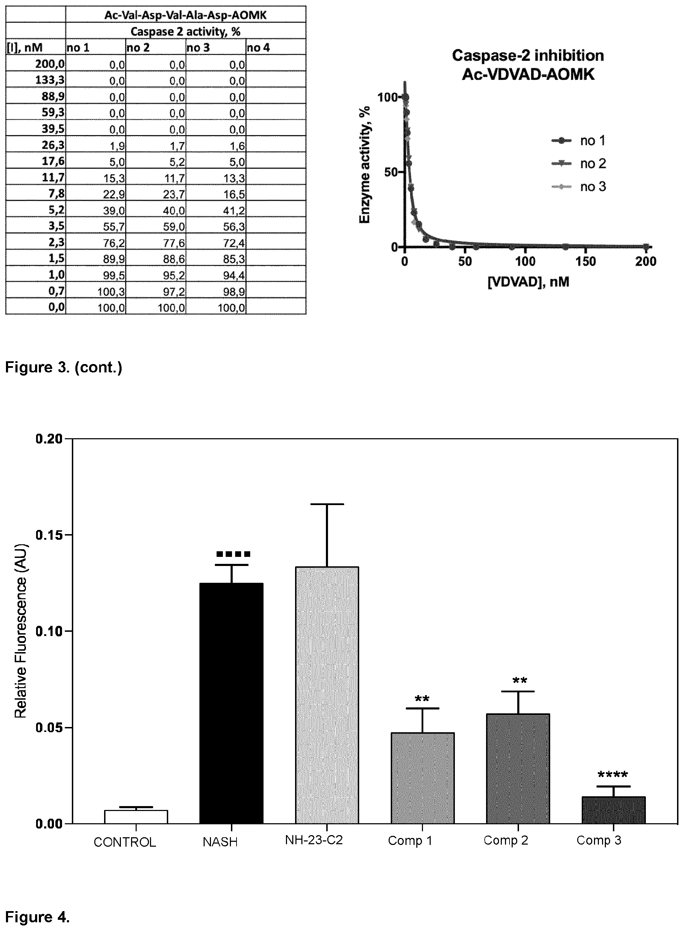

**Figure 3****.** Raw data for the determination of Ki and IC50 parameters for NH-23-C2 inhibitor towards caspase-2. The Ki parameter was measured using the Morrison equation (Copeland, 2000). NH-ldc-hGlu-Thr(Bzl)-Ser-Asp-ACC was used as ACC-fluorogenic substrate for caspase-2. The ACC fluorescence was monitored using 355 nm (excitation) and 460 nm (emission) wavelengths. The Ki parameter was calculated from Morrison equation, and the IC50 parameter was calculated using the formula: IC50 = Ki × (1+[S]/Km], where the [S] is substrate concentration used in the assay and Km is a Michaelis-Menten constant for a substrate. All the measurements were performed at least three times and the data was analyzed using GraphPad Prism 7 software.
**Figure 4****.** Lipid accumulation in HepG2 cells. Intracellular lipid accumulation was measured as Relative Fluorescence (Fluorescence493, BODIPY / Fluorescence503, DAPI). Results are expressed as Average± SEM (n=10). 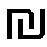 p<0,0001 vs control**p<0,05, ***p<0,001 vs NASH

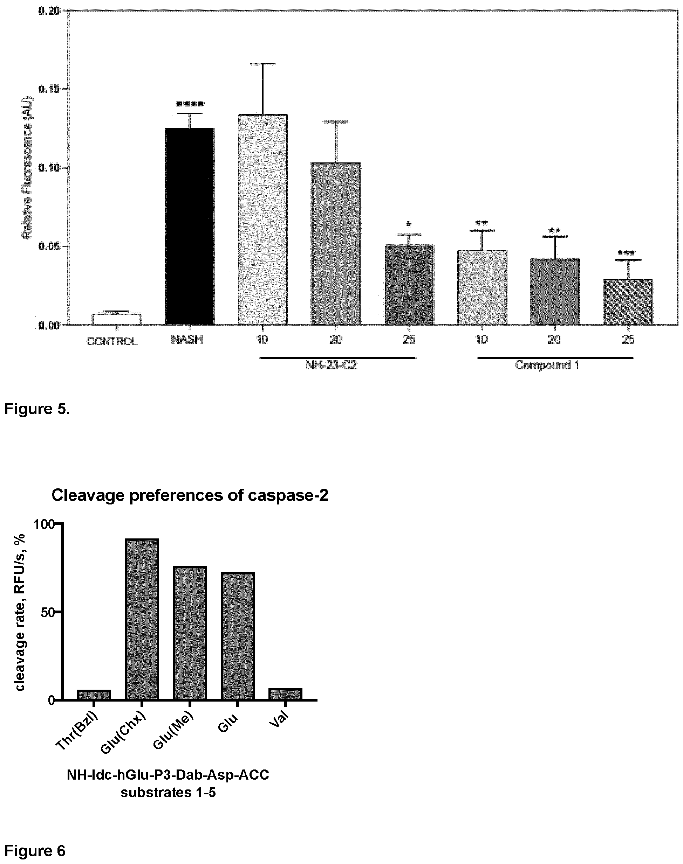

**Figure 5****.** Lipid accumulation in HepG2 cells. NH-23-C2 and Compound 1 were used at 10, 20 and 25mM. Intracellular lipid accumulation was measured as Relative Fluorescence (Fluorescence493, BODIPY/Fluorescence503, DAPI). Results are expressed as Average± SEM (n=10). 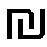 p<0,0001 vs control, ** p<0,05, ***p<0,001 vs NASH.
**Figure 6****.** Substrate preferences of recombinant caspase-2 tested towards 5 fluorogenic substrates with the general formula of NH-ldc-hGlu-P3-Dab-Asp-ACC, where P3 is Thr(Bzl), Glu(Chx), Glu, or Val. The cleavage rate of the best substrate (expressed as RFU/s, relative fluoresce units per second) was set to 100%, and the cleavage rates of other substrates were adjusted accordingly. Substrate concentration was 10□M, and caspase 2 concentration was 10 nM.

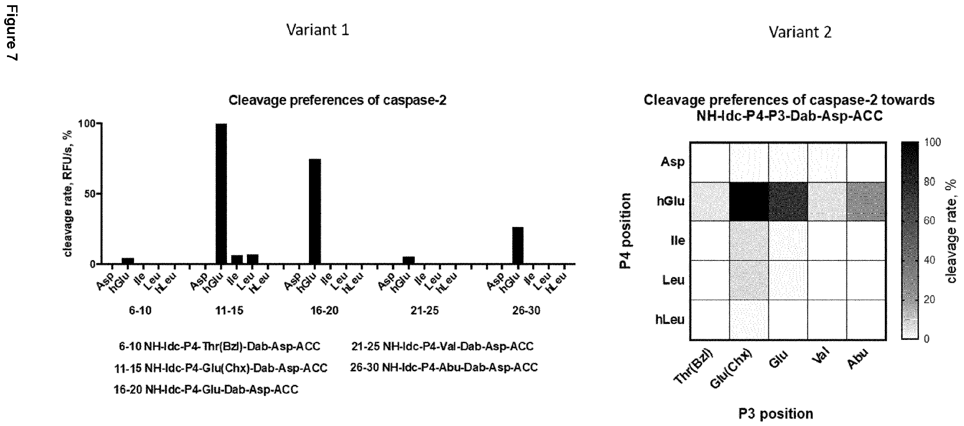

**Figure 7****.** Substrate preferences of recombinant caspase-2 tested towards 25 fluorescent substrates with the general formula of NH-ldc-P4-P3-Dab-Asp-ACC, where P4 is Asp, hGlu, Ile, Leu or hLeu and P3 is Thr(Bzl), Glu(Chx), Glu, Val, or Abu. Results are presented as a bar chart (variant 1) or as a heap map (variant 2). The cleavage rate of the best substrate (expressed as RFU/s, relative fluoresce units per second) was set to 100%, and the cleavage rates of other substrates were adjusted accordingly. Substrate concentration was 10µM, and caspase 2 concentration was 10 nM.

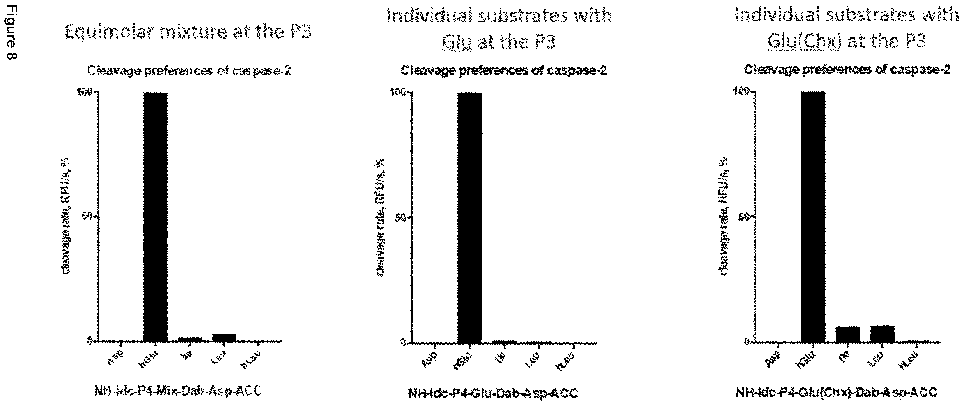

**Figure 8****.** Analysis of subsites cooperativity of caspase-2 at P4 and P3 positions using five combinatorial fluorogenic substrates with equimolar mixture of natural amino acids at the P3 position (NH-ldc-P4-Mix-Dab-Asp-ACC, left) and ten individual fluorogenic substrates with defined amino acids at the P3 position: glutamic acid - Glu (NH-ldc-P4-Glu-Dab-Asp-ACC, middle) or glutamic acid cyclohexyl ester - Glu(Chx) (NH-ldc-P4-Glu(Chx)-Dab-Asp-ACC, right). The cleavage rate of the best substrate from each series (expressed as RFU/s, relative fluoresce units per second) was set to 100%, and the cleavage rates of other substrates within the series were adjusted accordingly. The concentration of individual substrate was 10 µM, and the concentration of combinatorial substrates was 100 µM. Caspase 2 concentration was 10 nM.

### DETAILED DESCRIPTION OF THE INVENTION

In the context of the present invention, the following terms have the meaning detailed below:
The term "C₁₋₆ alkyl" refers to a linear or branched hydrocarbon chain radical consisting of carbon and hydrogen atoms, containing no unsaturation, having between 1 and 6, preferably between 1 and 3 ("C₁₋₃ alkyl"), more preferably 1 or 2 ("C₁₋₂ alkyl"), carbon atoms and which is attached to the rest of the molecule by a single bond, including for example and in a non-limiting sense, methyl, ethyl, n-propyl, i-propyl, n-butyl, t-butyl, n-pentyl, etc. Preferably "alkyl" refers to methyl or ethyl.

The term "C₃₋₇ cycloalkyl" refers to a saturated or partially saturated mono- or bicyclic aliphatic group having between 3 and 7, preferably between 3 and 6 carbon atoms which is bound to the rest of the molecule by means of a single bond, including for example and in a non-limiting sense, cyclopropyl, cyclohexyl or cyclopentyl.

The term "C₆₋₁₀ aryl" refers to an aromatic group having between 6 and 10, preferably 6 or 10 carbon atoms, comprising 1 or 2 aromatic nuclei, including for example and in a non-limiting sense, phenyl, naphthyl, etc. Preferably "aryl" refers to phenyl.

The term "halogen" refers to bromo, chloro, iodo or fluoro.

The term "C₁-C₆ haloalkyl" refers to an alkyl group as defined above wherein at least one of the hydrogen atoms has been replaced by a halogen atom such as, for example CF₃, CCl₃, CHF₂, CH₂F, CF₂CF₃.

The term "C₁₋₆ alkoxyl" refers to a group of formula -O-C₁₋₆ alkyl, wherein C₁₋₆ alkyl is an alkyl group as defined above, preferably C₁₋₃ alkyl. Examples of C₁₋₆ alkoxyl include methoxy, ethoxy, propoxy, iso-propoxy, butoxy, tert-butoxy, iso-butoxy and sec-butoxy, preferably methoxy.

The term "(C₆-C₁₀)aryl(C₁-C₆)alkyl" refers to an aryl group as defined above which is attached to the rest of the molecule through an alkyl group as defined above. Preferably, the (C₆-C₁₀)aryl(C₁-C₆)alkyl is a (C₆)aryl(C₁-C₃)alkyl, such as benzyl.

"5- to 10-membered heterocyclyl" refers to a stable 5- to 10-membered ring radical, preferably a 5- or 6-membered ring, which consists of carbon atoms and from one to five, preferably from 1 to 4, heteroatoms selected from the group consisting of nitrogen, oxygen, and sulphur and which can be partially or fully saturated. For the purposes of this invention, the heterocycle can be a monocyclyl or bicyclyl ring system. Examples of such heterocycles include, but are not limited to, pyrrolidine, piperidine, tetrahydropyridine, piperazine, morpholine, thiomorpholine, diazepane, tetrahydrofuran, tetrahydropyran, octahydro-pyrrolopyrazine.

"5- to 10-membered heteroaryl" refers to a stable 5- to 10-membered aromatic ring radical, preferably a 5- or 6-membered aromatic ring, which consists of carbon atoms and from one to five, preferably from 1 to 4, heteroatoms selected from the group consisting of nitrogen, oxygen, and sulphur. For the purposes of this invention, the heteroaryl can be a monocyclyl or bicyclyl ring system. Examples of such heteroaryl include, but are not limited to, thiophene, furan, pyrrole, thiazole, oxazole, isothiazole, isooxazole, imidazole, pyrazole, triazole, oxadiazole, thiadiazole, tetrazole, tetrazole oxide, oxadiazolone, pyridine, pyrimidine, dihydroindolone, benzimidazole, benzothiazole, benzofuran, indole, purine, quinoline.

As understood in this technical area, there can be a certain degree of substitution on the previously defined radicals. Thus, there can be substitution in any of the groups of the present invention. The references of the present document to substituted groups in the groups of the present invention indicate that the specified radical can be substituted in one or more available positions by one or more substituents. Said substituents include, for example and in a non-limiting sense, halogen, C₁₋₆ alkyl, C₃₋₇ cycloalkyl, C₁₋₆ haloalkyl, C₆₋₁₀ aryl, (C₆₋₁₀)aryl(C₁₋₆)alkyl, 5- to 10-membered heterocyclyl, 5- to 10-membered heteroaryl, -CN, -NO₂, -OR, -SR, -C(O)R, -C(O)OR, -OC(O)R, - C(O)NR₂, -NR₂ and -SO₂R; wherein each R is independently selected from H, C₁₋₆ alkyl, C₁₋₆ haloalkyl, C₃₋₇ cycloalkyl, C₆₋₁₀ aryl, (C₆₋₁₀)aryl(C₁₋₆)alkyl, 5- to 10-membered heterocyclyl, and 5- to 10-membered heteroaryl.

The compounds of the invention may be in the form of salts, solvates or stereoisomers, preferably pharmaceutically acceptable salts, solvates or stereoisomers.

As already indicated, the present invention also provides "salts" of the compounds described herein. By way of illustration, said salts can be acid addition salts, base addition salts or metal salts, and can be synthesized from the parent compounds containing a basic or acid moiety by means of conventional chemical processes known by the person skilled in the art. See, generally, G. S. Paulekuhn, et al., "Trends in Active Pharmaceutical Ingredient Salt Selection based on Analysis of the Orange Book Database", J. Med. Chem., 2007, 50: 6665-72, S. M. Berge, et al., "Pharmaceutical Salts", J Pharm Sci., 1977, 66:1-19, and Handbook of Pharmaceutical Salts, Properties, Selection, and Use, Stahl and Wermuth, Eds., Wiley-VCH and VHCA, Zurich, 2002. Such salts are generally prepared, for example, by reacting the free acid or base forms of said compounds with a stoichiometric amount of the suitable base or acid in water or in an organic solvent or in a mixture of the two. Non-aqueous media such as ether, ethyl acetate, ethanol, acetone, isopropanol or acetonitrile are generally preferred. Illustrative examples of acid addition salts include inorganic acid addition salts such as, for example, hydrochloride, hydrobromide, hydroiodide, sulfate, pyrosulfate, bisulfate, sulfite, bisulfite, nitrate, phosphate, monohydrogen-phosphate, dihydrogenphosphate, meta-phosphate, pyrophosphate, etc., organic acid addition salts such as, for example, acetate, maleate, fumarate, citrate, oxalate, succinate, tartrate, malate, mandelate, methanesulfonate, p-toluenesulfonate, camphorsulfonate, propionates, decanoates, caprylates, acrylates, formates, isobutyrates, caproates, heptanoates, propiolates, malonates, succinates, suberates, sebacates, butyne-1,4-dioates, hexyne-1,6-dioates, benzoates, chlorobenzoates, methyl benzoates, dinitrobenzoates, hydroxybenzoates, methoxybenzoates, phthalates, sulfonates, xylenesulfonates, phenylacetates, phenylpropionates, phenylbutyrates, lactates, y-hydroxybutyrates, glycolates, propanesulfonates, naphthalene-1-sulfonates, naphthalene-2-sulfonates, etc. Illustrative examples of base addition salts include inorganic base salts such as, for example, ammonium salts and organic base salts such as, for example, ethylenediamine, ethanolamine, *N*,*N*-dialkylenethanolamine, triethanolamine, glutamine, amino acid basic salts, etc. Illustrative examples of metal salts include, for example, sodium, potassium, calcium, magnesium, aluminum and lithium salts.

The term "solvate" according to this invention is to be understood as meaning any form of the active compound according to the invention which has another molecule (most likely a polar solvent) attached to it via non-covalent bonding. Examples of solvates include hydrates and alcoholates. Solvation methods are generally known in the state of the art.

As used herein, the term "stereoisomer" is a general term for all isomers of individual molecules that differ only in the orientation of their atoms in space. Therefore, stereoisomer compounds are molecules that are non-superimposable mirror images of each other and include enantiomers and diastereomers.

The term "chiral center" refers to a carbon atom to which four different groups are attached.

The terms "enantiomer" and "enantiomeric form" refer to one of the two stereoisomers of a compound that are mirror images of each other that are non-superimposable. Enantiomer compounds are optically active, wherein one enantiomer rotates the plane of polarized light in one direction and the other one rotates the plane of polarized light in the opposite direction and form a racemic compound when present in equal quantities.

The term "racemic" or "racemate" refers to a mixture that has equal amounts of enantiomers and which mixture is optically inactive.

The terms "diastereomer" and "diastereomeric form" refer to stereoisomers of a compound with more than one chiral center that are not mirror images of one another.

As will be understood, the terms enantiomerically enriched or diastereomerically enriched describes a mixture of two enantiomers or a mixture of diastereomers in which one enantiomer or diastereomer is present in greater amount than the other enantiomer or diastereomer.

The compounds of the invention have chiral centers and therefore can exist in different stereoisomeric forms, such as enantiomeric or diastereomeric forms. Thus, any given compound referred to herein is intended to represent any one of a racemate, one or more enantiomeric forms and one or more diastereomeric forms. All the stereoisomers including enantiomers and diastereoisomers of the compounds referred to herein, and mixtures thereof (including racemic mixtures, enantiomerically enriched mixtures and diastereomerically enriched mixtures), are considered within the scope of the present invention.

The term "pharmaceutically acceptable" relates to molecular entities and compositions being physiologically tolerable and normally not causing an allergic reaction or similar adverse reaction, such as gastric discomfort, dizziness and the like, when they are administered to a human being. Preferably, as used in this description, the term "pharmaceutically acceptable" means approved by a governmental regulatory agency or listed in the US pharmacopoeia or another generally recognized pharmacopoeia for use in animals, and more particularly in humans.

A "fluorophore", as used herein refers to a molecule or moiety that can re-emit light upon light excitation.

As used herein, the term "warhead" refers to a moiety present on a compound of the present invention which is capable of covalently binding with an active site of the target enzyme (Caspase-2), thereby achieving irreversible inhibition effects. One of ordinary skill in the art will recognize that certain reactive functional groups can act as warheads. It is noted that "Covalently binding" is not equal to "irreversible inhibition". For example aldehydes bind covalently but reversibly. Even some AOMKs can act as reversible inhibitors:
1) Brady KD. Bimodal inhibition of caspase-1 by aryloxymethyl and acyloxymethyl ketones. Biochemistry. 1998; 37:8508-15.
2) Brady KD, Giegel DA, Grinnell C, Lunney E, Talanian RV, Wong W, et al. A catalytic mechanism for caspase-1 and for bimodal inhibition of caspase-1 by activated aspartic ketones. Bioorg Med Chem. 1999; 7:621-31.

Poreba et al. Caspase selective reagents for diagnosing apoptotic mechanisms, CDD 2019.

### Compounds of the invention

The present specification exemplifies potent and selective caspase-2 ligands. In particular, the present application exemplifies compounds of formula (I): 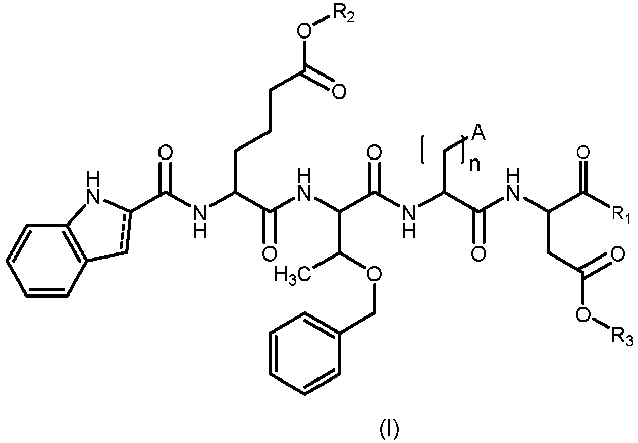 or a salt, solvate or stereoisomer thereof;
wherein
R2 and R3 can each be independently H, and R2 and R₃ can also be absent so to result in the deprotonated form CO(O⁻),
wherein
n is 1, 2, or 3;
A is an amine group; and
R1 is a reactive group.

In the examples of the present specification, it is therein shown that even minor modifications at P2 of compounds of formula (I) have a profound impact on binding affinity of the resulting analogues (see example 2). In this sense, it is clear that while a replacement of Ser-> Lys, Orn or Dab is tolerated by caspase 2, this is not the case for caspase 3 and caspase 8 (see tables 3-5). This unexpected result leads to an increased selectivity of those compounds towards caspase-2, especially the Dab analogue being 5 fold more selective over caspase 3 and over 20 fold over caspase 8 (see tables 6-7).

Therefore, the present specification discloses specific ligands of formula (I) having a free primary amine group at P2. Specifically, the present application exemplifies that peptides corresponding to Idc-hGlu-Thr(Bzl)-[Lys/Orn/Dab]-Asp-OR1 are indeed potent and selective ligands for caspase 2 (see examples 2 and 3).

However, peptide-based therapeutics show rapid in vivo proteolysis, poor oral bioavailability and tend to display poor pharmacokinetics. More precisely, peptide-based caspase inhibitors have limited clinical applications due to their poor pharmacokinetic properties, including poor oral bioavailability, cell permeability, and metabolic stability.

Over the last years, several concepts and strategies have been reported to improve peptidogenic inhibitors properties including incorporation of non-natural aminoacids, methylations of carboxy groups, fluorine substituents, designed salt-bridges (S. Xiong, J. Virol. 2017, 91), the introduction of D-amino acids (B. D. Welch, J. Virol. 2010, 84), backbone crosslinks (O.Bolarinwa, Org. Biomol. Chem. 2018, 16, 7878-7882.), hydrogen-bond-surrogates (D. Wang. Chem. Int. Ed. 2008, 47, 1879-1882), foldamers (W. S. Horne, Proc. Natl. Acad. Sci. USA 2009, 106, 14751-14756.), cyclization (M. K. Lee, Exp. Mol. Med. 2006, 38, 18-26.), multimerization (C. Whang, J. Med. Chem. 2022, 65, 4, 2809-2819), conjugation to small molecules (M. Ferrer, Nat. Struct. Biol. 1999, 6, 953-960), or to different classes of lipids (Y. Zhu, H. Chong, J. Virol. 2019, 93, e02312-02318), as well as PEGylation (C. Wang, Viruses 2019, 11, 811) or dual-targeting inhibitors (D. Wensel, J. Virol. 2019, 93, e00907-00919). The obtained derivatives can display increased activity but more importantly, show improved metabolic stability compared to wild-type peptides.

Methylation of carboxy group of the sidechain of aminoacids confers beneficial properties on the peptide, such as increase in solubility in aqueous and/or- organic solvents, resistance to proteolytic degradation, diffusion cross membranes and increased oral bioavailability (Gordon DJ, J. Pept. Res. 2002;60:34-55, Adessi C, Biol. Chem. 2003;278(16):13905-13911., Kokkoni N. Biochemistry. 2006;45:9906-9918). Thus, in the present invention, we have methylated the side chain of the aspartic acid (R₃ in formula I) and of the homo-glutamic acid (R₂ in formula I) as this is expected to increase cell membrane permeability as successfully exemplified in a common inhibitor used to treat caspases in cell-based experiments - Z-VAD-fmk. In this sense and in the case of Z-VAD-fmk, methylation of the aspartic acid side chain significantly increased cell permeability without affecting the activity of the molecule.

On the other hand, decoration of organic molecules with fluorine substituents is a widely used strategy to improve small molecule drugs, owing to fluorine's capacity to productively affect molecular properties, such as conformation, pKa values of neighboring functional groups, and hydrophobicity. Often, this leads to improved interactions with proteins, enzymes, receptors, or membranes, thereby improving pharmacological properties, cell-permeability, bioavailability, and activity (S. Purser, Chem. Soc. Rev. 2008, 37, 320-330.). Moreover, due to the remarkable stability of the fluorine covalent bond, these molecules often show improved metabolic stability.

With the advent of easy to access fluorine-containing amino acids (J. Moschner, Chem. Rev. 2019, 119, 10718-10801; J. Leppkes, J. Fluorine Chem. 2020, 232, 109453.), these building blocks sparked a renewed interest for drug design (H. Mei, Eur. J. Med. Chem. 2020, 186, 111826.) and peptide and protein engineering. Selective introduction of fluorine-containing amino acids into peptides can modulate distinct pharmacokinetic and physicochemical functionalities of the resulting biopolymers, including structure, folding or stability towards thermal and chemical denaturation. Furthermore, it can improve metabolic stability, biological activity, cell-permeability and bioavailability of peptide-based drugs (G. Akcay, J. Fluorine Chem. 2009, 130, 1178-1182; M. Salwiczek, Chem. Soc. Rev. 2012, 41, 2135-2171; E. N. G. Marsh, Acc. Chem. Res. 2014, 47, 2878-2886; A. A. Berger, Acc. Chem. Res. 2017, 50, 2093-2103; S. Huhmann, Eur. J. Org. Chem. 2018, 3667-3679; R. Smits, Curr. Top. Med. Chem. 2006, 6, 1483-1498).

In this patent we sought to introduce fluorinated amino acid analogues and methylations in the carboxy groups of the amino acid side chains into a compound that targets caspase-2 to improve its druggability properties.

Therefore, in the present invention we herein provide compounds of general formula (II): 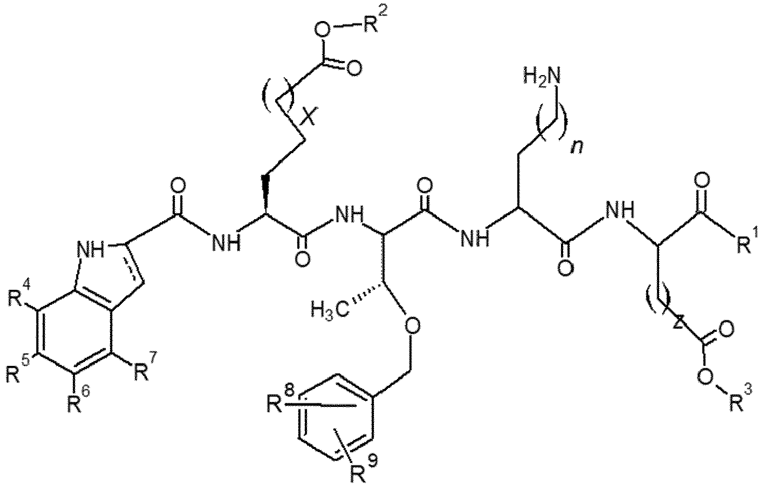
or a salt, solvate or stereoisomer thereof;
wherein: 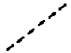 (the dotted line) in the heterocyclic ring of position P5 represents a bond which maybe present or absent, when present it forms a double bond in combination with the single bond already present;
R₂ and R₃ are independently chosen from a hydrogen or a C₁₋₄ alkyl including branched alkyl and cycloalkyl;
   R₄, R₅, R₆ and R₇ are each independently chosen from hydrogen, halogen or C₁₋₄alkyl including branched alkyl and cycloalkyl, wherein each C₁₋₄ alkyl is optionally substituted with 1 -3 halogen atoms;
   x, and z are each independently chosen from 0, 1, or 2; n is selected from 0, 1, 2, or 3;
   R₈ and R₉ are independently chosen from hydrogen, halogen or C1-4 alkyl including branched alkyl and cycloalkyl, wherein each C1-4 alkyl is optionally substituted with 1 -3 halogen atoms.
   R₁ is a chemically reactive group. Chemically reactive groups can be incorporated into drug molecules to confer certain advantages as described by Gehringer et al (J. Med. Chem. 2019, 62, (12) 5673-5724, particularly in the field of enzyme inhibition where the group can form a covalent bond between the drug and the enzyme thereby enhancing the inhibition profile. R1 can be a fluorophore (to be use in the context of substrates), or a warhead (to be use in the context of inhibitors).

It is noted that in the case of substrates (such as those comprising the fluorophores) exemplified below, R1 is linked to the P1 subunit through a covalent peptide bond between the amino group of the R1 subunit (or any other group indicated as W) and the a-carboxyl group of the P1 amino acid. Preferably, R1 is a moiety such as a fluorophore as ACC of formula:

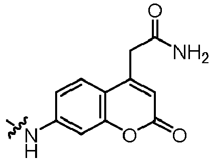

More particularly, R₁ can be a moiety such as a fluorophore (to be use in the context of substrates) selected from a group of formula: 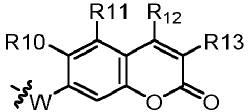 wherein
W is selected from -NH- and -O-,
R10-R13 are independently selected from H, C₁₋₆ alkyl and C₃₋₇ cycloalkyl, Aryl, heteroaryl, CF₃, -CH₂COOH, -CH₂CONHR14, and CH₂OR14, and
R14 is selected from H, C1-6 alkyl and C3-7 cycloalkyl.

Examples of R1 moieties as fluorophores can be selected from any of the following list:

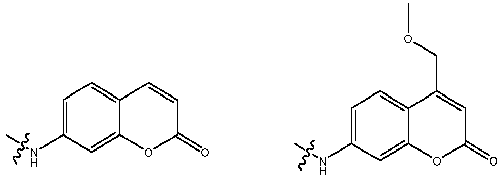

Other examples of potentially useful moieties as fluorophores can be selected from the list consisting of: 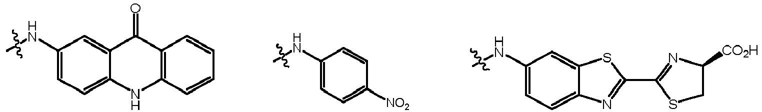 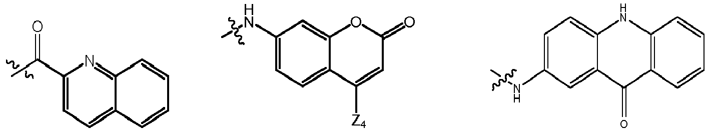 or

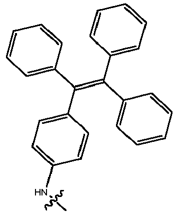

In the context of the present invention, a fluorophore (or fluorochrome, similarly to a chromophore) is a fluorescent chemical compound that can re-emit light upon light excitation. Fluorophores or substrates typically contain several combined aromatic groups, or planar or cyclic molecules with several π bonds. Fluorophores are notably used to stain tissues, cells, or materials in a variety of analytical methods, i.e., fluorescent imaging and spectroscopy. wherein W of the above formula should represent a -NH-such as in ACC. Further fluorophores in R₁ can be selected from common fluorophores including Indo-1, Ca saturated Indo-1 Ca2+ Cascade Blue BSA pH 7.0 Cascade Blue
LysoTracker Blue Alexa 405 LysoSensor Blue pH 5.0 LysoSensor Blue DyLight 405 DyLight 350 BFP (Blue Fluorescent Protein) Alexa 350
7-Amino-4-methylcoumarin pH 7.0, AminoCoumarin AMCA conjugateCoumarin 7-Hydroxy-4-methylcoumarin7-Hydroxy-4methylcoumarin pH 9.0
6,8-Difluoro-7-hydroxy-4-methylcoumarin pH 9.0 Hoechst 33342 Pacific Blue Hoechst 33258 Hoechst 33258-DNA Pacific Blue antibody conjugate pH 8.0 PO-PRO-1PO-PRO-1-DNA POPO-1 433 nm POPO-1-DNA DAPI-DNA DAPI Marina Blue SYTOX Blue-DNA CFP (Cyan Fluorescent Protein) eCFP (Enhanced Cyan Fluorescent Protein) 1-Anilinonaphthalene-8-sulfonic acid (1,8-ANS) Indo-1, Ca free 1,8-ANS (1-Anilinonaphthalene-8-sulfonic acid)
BO-PRO-1-DNA BOPRO-1 BOBO-1-DNA SYTO 45-DNA
evoglow-Pp1 evoglow-Bs1 evoglow-Bs2 Auramine O DiO LysoSensor Green pH 5.0 Cy 2 LysoSensor Green Fura-2, high Ca Fura-2 Ca2+sup> SYTO 13-DNA YO-PRO-1-DNA YOYO-1-DNA eGFP (Enhanced Green Fluorescent Protein) LysoTracker Green GFP (S65T) BODIPY FL, MeOH Sapphire
BODIPY FL conjugate MitoTracker Green MitoTracker Green FM Fluorescein 0.1 M NaOH Calcein pH 9.0 Fluorescein pH 9.0 Calcein
Fura-2, no Ca Fluo-4 FDA DTAF Fluorescein
   Fluorescein antibody conjugate pH 8.0 CFDA FITC Alexa Fluor 488 hydrazide-water DyLight 488 5-FAM pH 9.0 FITC antibody conjugate pH 8.0 Alexa 488
Rhodamine 110 Rhodamine 110 pH 7.0 Acridine Orange Alexa Fluor 488 antibody conjugate pH 8.0 BCECF pH 5.5 PicoGreendsDNA quantitation reagent SYBR Green I Rhodaminen Green pH 7.0 CyQUANT GR-DNA
NeuroTrace 500/525, green fluorescent Nissl stain-RNA DansylCadaverine
Rhodol Green antibody conjugate pH 8.0 Fluoro-EmeralNissl Fluorescein dextran pH 8.0 Rhodamine Green 5-(and-6)-Carboxy-2', 7' dichlorofluorescein pH 9.0 DansylCadaverine, MeOH eYFP (Enhanced Yellow Fluorescent Protein)
Oregon Green 488 Oregon Green 488 antibody conjugate pH 8.0 Fluo-3
BCECF pH 9.0 SBFI-Na+
   Indo-1, Ca saturated Indo-1 Ca2+ Cascade Blue BSA pH 7.0 Cascade Blue
LysoTracker Blue Alexa 405 LysoSensor Blue pH 5.0 LysoSensor Blue DyLight 405 DyLight 350 BFP (Blue Fluorescent Protein) Alexa 350
7-Amino-4-methylcoumarin pH 7.0, AminoCoumarin AMCA conjugateCoumarin 7-Hydroxy-4-methylcoumarin7-Hydroxy-4methylcoumarin pH 9.0
6,8-Difluoro-7-hydroxy-4-methylcoumarin pH 9.0 Hoechst 33342 Pacific Blue Hoechst 33258 Hoechst 33258-DNA Pacific Blue antibody conjugate pH 8.0 PO-PRO-1PO-PRO-1-DNA POPO-1 433 nm POPO-1-DNA DAPI-DNA DAPI Marina Blue SYTOX Blue-DNA CFP (Cyan Fluorescent Protein) eCFP (Enhanced Cyan Fluorescent Protein) 1-Anilinonaphthalene-8-sulfonic acid (1,8-ANS) Indo-1, Ca free 1,8-ANS (1-Anilinonaphthalene-8-sulfonic acid)
BO-PRO-1-DNA BOPRO-1 BOBO-1-DNA SYTO 45-DNA
evoglow-Pp1 evoglow-Bs1 evoglow-Bs2 Auramine O DiO LysoSensor Green pH 5.0 Cy 2 LysoSensor GreenLysoSensor Yellow pH 9.0
   Indo-1, Ca saturated Indo-1 Ca2+ Cascade Blue BSA pH 7.0 Cascade Blue
LysoTracker Blue Alexa 405 LysoSensor Blue pH 5.0 LysoSensor Blue DyLight 405 DyLight 350 BFP (Blue Fluorescent Protein) Alexa 350
7-Amino-4-methylcoumarin pH 7.0, AminoCoumarin AMCA conjugateCoumarin 7-Hydroxy-4-methylcoumarin7-Hydroxy-4methylcoumarin pH 9.0
6,8-Difluoro-7-hydroxy-4-methylcoumarin pH 9.0 Hoechst 33342 Pacific Blue Hoechst 33258 Hoechst 33258-DNA Pacific Blue antibody conjugate pH 8.0 PO-PRO-1PO-PRO-1-DNA POPO-1 433 nm POPO-1-DNA DAPI-DNA DAPI Marina Blue SYTOX Blue-DNA CFP (Cyan Fluorescent Protein) eCFP (Enhanced Cyan Fluorescent Protein) 1-Anilinonaphthalene-8-sulfonic acid (1,8-ANS) Indo-1, Ca free 1,8-ANS (1-Anilinonaphthalene-8-sulfonic acid)
BO-PRO-1-DNA BOPRO-1 BOBO-1-DNA SYTO 45-DNA
evoglow-Pp1 evoglow-Bs1 evoglow-Bs2 Auramine O DiO LysoSensor Green pH 5.0 Cy 2 LysoSensor Green.

On the other hand, in the case of in inhibitors, P1 is linked to R1 through a covalent bond between the a-carboxyl group of the P1 amino acid and the R1 moiety, wherein R1 could be a large number of moieties such as acyloxymethyl ketone (in AOMK) or just a simple hydrogen atom (in aldehydes).

Thus, in another embodiment, R1 can be a warhead, in particular, R1 can be a warhead (to be use in the context of inhibitors) selected from the group consisting of: 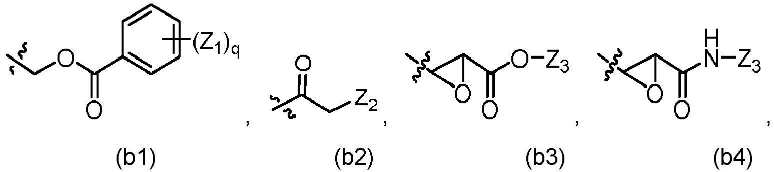 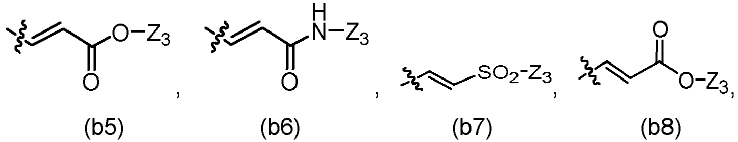 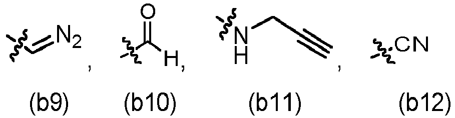 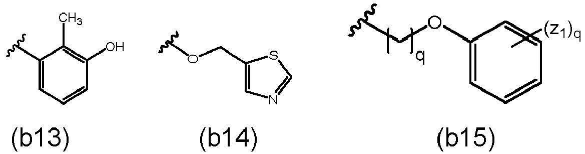 wherein
q is 0, 1, 2, 3, 4 or 5,
each Z₁ is independently selected from C₁₋₆ alkyl, halogen, -CN, -NO₂ and C₁₋₆ alkoxyl,
Z₂ is halogen, and
Z₃ is selected from C₁₋₆ alkyl, C₆₋₁₀ aryl and (C₆₋₁₀)aryl(C₁₋₆)alkyl,
or a salt, solvate or stereoisomer thereof.

Specific examples of warheads useful in the present invention can be selected from any of the following (all of the following compounds are directly bound to the a-COOH group of the P1 subunit):

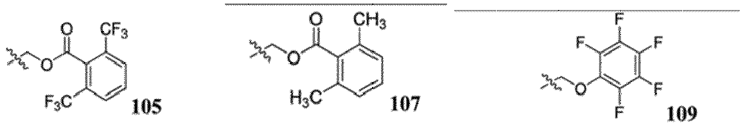

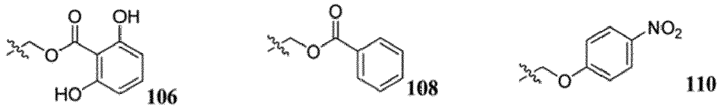

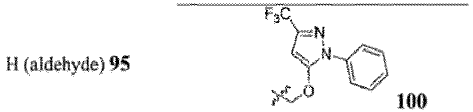

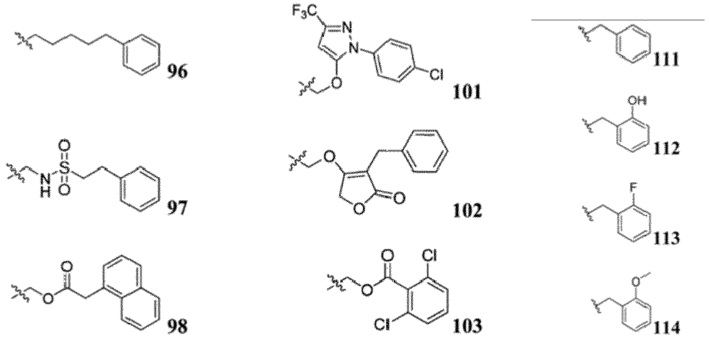

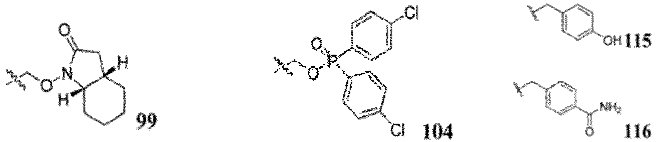

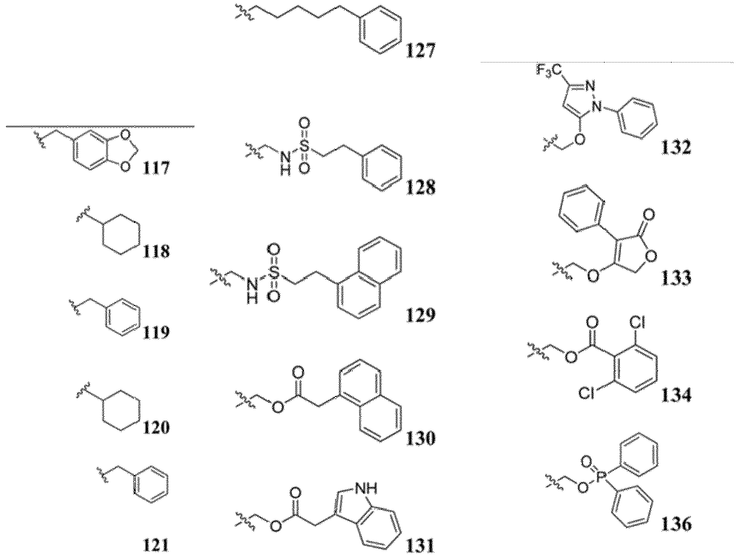

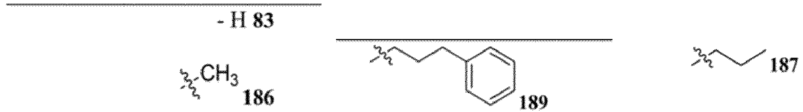

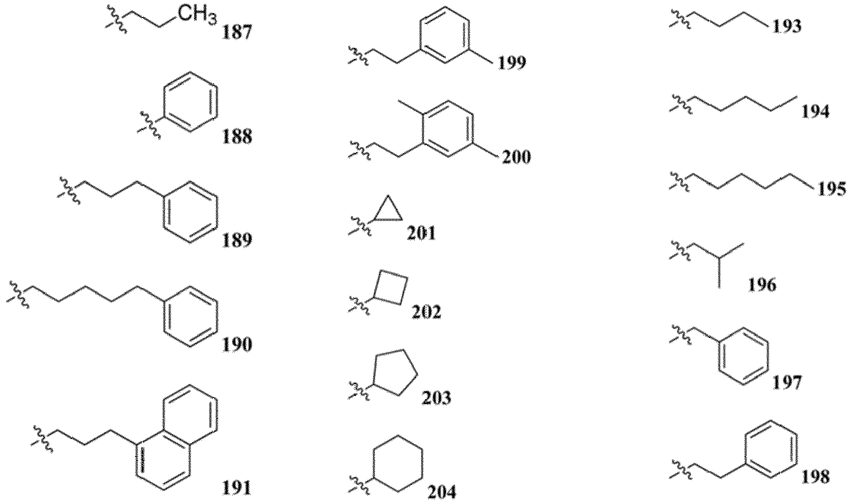

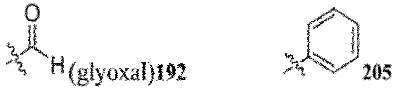

Preferably, R1 is a warhead of formula: 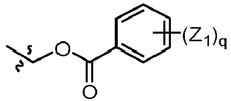 wherein
q is 0, 1, 2, 3 or 4, and
each Z₁ is independently selected from C₁₋₆ alkyl, halogen, -CN and C₁₋₆ alkoxyl.
   In an embodiment, Z₁ is independently selected from halogen, methyl and -OMe; preferably from F, Cl and Me (methyl).
   In a further embodiment, R₁ is selected from a group of formula 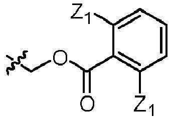 wherein each Z₁ is independently selected from C₁₋₆ alkyl, halogen, -CN and C₁₋₆ alkoxyl; preferably from halogen, methyl and -OMe; more preferably from F, Cl and Me (methyl).

In a preferred embodiment, R₁ has formula

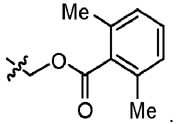

In another preferred embodiment, R₁ is represented by the following formula: 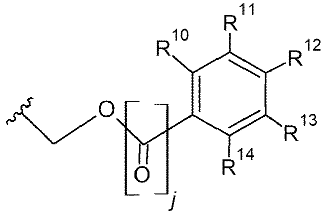 wherein
j is 0 or 1;
R₁₀, R₁₁, R₁₂, R₁₃ and R₁₄ are each independently selected from hydrogen, halogen or a C₁₋₄ alkyl including branched alkyl and cycloalkyl, phenyl, phenoxy, nitro, nitrile, PhCO, NHAc, wherein each alkyl is optionally substituted with 1 -3 halogen atoms.

It is noted that, in certain embodiments, either R10 and R11, R14 and R13, R13 and R12, or R11 and R12 together form an additional aromatic ring, preferably a fused phenyl ring forming a naphthyl group

In another preferred embodiment, R₁ incorporates the P1 group to form a group having the following formula:

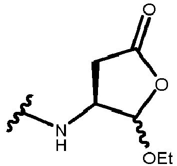

In another embodiment, in the present invention we herein provide compounds of general formula (II), or a salt, solvate or stereoisomer thereof, wherein: 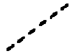 (the dotted line) in the heterocyclic ring of position P5 represents a bond which maybe present or absent, when present it forms a double bond in combination with the single bond already present;
R₄, R₅, R₆ and R₇ are each independently chosen from hydrogen, a halogen such as a fluorine or a trifluoromethyl;
x, and z are each 1;
n is 0, 1, 2 or 3;
R₈ and R₉ are each independently chosen from hydrogen, a halogen such as a fluorine or a trifluoromethyl;
R₂ and R₃ are independently chosen from a hydrogen or a C₁₋₄ alkyl, preferably methyl or ethyl; and
R₁ is a chemically reactive group as defined above.

In another embodiment, in the present invention we herein provide compounds of general formula (II), or a salt, solvate or stereoisomer thereof,
wherein: 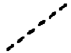 (the dotted line) in the heterocyclic ring of position P5 represents a bond which maybe present or absent, when present it forms a double bond in combination with the single bond already present;
R₄, R₅, R₆ and R₇ are each independently chosen from hydrogen or a halogen such as a fluorine;
x, and z are each 1;
n is 0, 1, 2 or 3;
R₈ and R₉ are each independently chosen from hydrogen, a halogen such as a fluorine or a trifluoromethyl;
R₂ and R₃ are independently chosen from hydrogen or methyl; and
R₁ is a chemically reactive group as defined above.

In another embodiment, in the present invention we herein provide compounds of general formula (II), or a salt, solvate or stereoisomer thereof,
wherein: 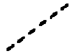 (the dotted line) in the heterocyclic ring of position P5 represents a bond which maybe present or absent, when present it forms a double bond in combination with the single bond already present;
R₄, and R₆ are each independently chosen from a halogen such as a fluorine and R₅ and R₇ are both hydrogen;
x, and z are each 1;
n is 0, 1, 2 or 3;
R₈ and R₉ are each independently chosen from hydrogen, a halogen such as a fluorine or a trifluoromethyl;
R₂ and R₃ are independently chosen from hydrogen or methyl; and
R₁ is a chemically reactive group as defined above.

In another embodiment, in the present invention we herein provide compounds of general formula (II), or a salt, solvate or stereoisomer thereof,
wherein: 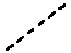 (the dotted line) in the heterocyclic ring of position P5 represents a bond which maybe present or absent, when present it forms a double bond in combination with the single bond already present;
R₄, and R₆ are each independently chosen from a halogen such as a fluorine and R₅ and R₇ are both hydrogen;
x, and z are each 1;
n is 0, 1, 2 or 3;
R₈ and R₉ are both hydrogen;
R₂ and R₃ are independently chosen from hydrogen or methyl; and
R₁ is a chemically reactive group as defined above.

In yet another embodiment, in the present invention we herein provide compounds of general formula (III), or a salt, solvate or stereoisomer thereof, 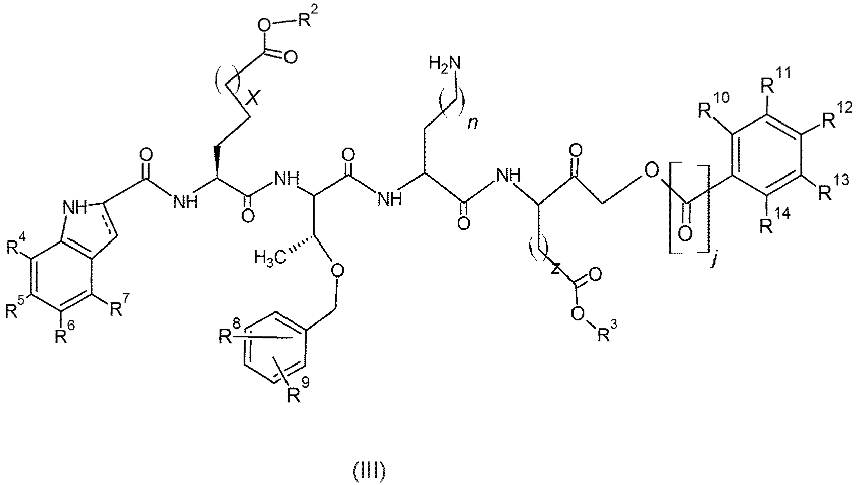 wherein 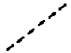
(the dotted line) in the heterocyclic ring of position P5 represents a bond which maybe present or absent, when present it forms a double bond in combination with the single bond already present;
R₂ and R₃ are independently chosen from a hydrogen or a C₁₋₄ alkyl including branched alkyl and cycloalkyl;
   R₄, R₅, R₆ and R₇ are each independently chosen from hydrogen, halogen or C₁₋₄ alkyl including branched alkyl and cycloalkyl, wherein each C₁₋₄ alkyl is optionally substituted with 1 -3 halogen atoms;
   x, and z are each independently chosen from 0, 1, or 2; n is 0, 1, 2 or 3;
   R₈ and R₉ are independently chosen from hydrogen, halogen or C1-4 alkyl including branched alkyl and cycloalkyl, wherein each C1-4 alkyl is optionally substituted with 1 -3 halogen atoms;
   j is 0 or 1; and
   R₁₀, R₁₁, R₁₂, R₁₃ and R₁₄ are each independently selected from hydrogen, halogen or a C1-4 alkyl including branched alkyl and cycloalkyl, phenyl, phenoxy, nitro, nitrile, PhCO, NHAc, wherein each alkyl is optionally substituted with 1 -3 halogen atoms.
   It is noted that, in certain embodiments, either R₁₀ and R₁₁, R₁₄ and R₁₃, R₁₃ and R₁₂, or R₁₁ and R₁₂ together form an additional aromatic ring, preferably a fused phenyl ring forming a naphthyl group.

In yet another embodiment, in the present invention we herein provide compounds of general formula (III), or a salt, solvate or stereoisomer thereof,
wherein: 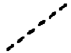
(the dotted line) in the heterocyclic ring of position P5 represents a bond which maybe present or absent, when present it forms a double bond in combination with the single bond already present;
   R₄, R₅, R₆ and R₇ are each independently chosen from hydrogen, a halogen such as a fluorine or a trifluoromethyl;
   x, and z are each 1;
   n is 0, 1, 2 or 3;
   R₈ and R₉ are each independently chosen from hydrogen, a halogen such as a fluorine or a trifluoromethyl;
   R₂ and R₃ are independently chosen from hydrogen or a C₁₋₄ alkyl, preferably methyl or ethyl;
j is 0 or 1; and
R₁₀, R₁₁, R₁₂, R₁₃ and R₁₄ are each independently selected from hydrogen, halogen or a C1-4 alkyl including branched alkyl and cycloalkyl, phenyl, phenoxy, nitro, nitrile, PhCO, NHAc, wherein each alkyl is optionally substituted with 1 -3 halogen atoms.
It is noted that, in certain embodiments, either R₁₀ and R₁₁, R₁₄ and R₁₃, R₁₃ and R₁₂, or R₁₁ and R₁₂ together form an additional aromatic ring, preferably a fused phenyl ring forming a naphthyl group.

In another embodiment, in the present invention we herein provide compounds of general formula (III), or a salt, solvate or stereoisomer thereof,
wherein: 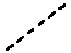
(the dotted line) in the heterocyclic ring of position P5 represents a bond which maybe present or absent, when present it forms a double bond in combination with the single bond already present;
   R₄, R₅, R₆ and R₇ are each independently chosen from hydrogen, a halogen such as a fluorine or a trifluoromethyl;
   x, and z are each 1;
   n is 0, 1, 2 or 3;
   R₈ and R₉ are each independently chosen from hydrogen, a halogen such as a fluorine or a trifluoromethyl;
   R₂ and R₃ are independently chosen from hydrogen or methyl;
j is 0 or 1; and
R₁₀, R₁₁, R₁₂, R₁₃ and R₁₄ are each independently selected from hydrogen, halogen or a C1-4 alkyl including branched alkyl and cycloalkyl, phenyl, phenoxy, nitro, nitrile, PhCO, NHAc, wherein each alkyl is optionally substituted with 1 -3 halogen atoms.
It is noted that, in certain embodiments, either R₁₀ and R₁₁, R₁₄ and R₁₃, R₁₃ and R₁₂, or R₁₁ and R₁₂ together form an additional aromatic ring, preferably a fused phenyl ring forming a naphthyl group.

In yet another embodiment, in the present invention we herein provide compounds of general formula (IV), or a salt, solvate or stereoisomer thereof, 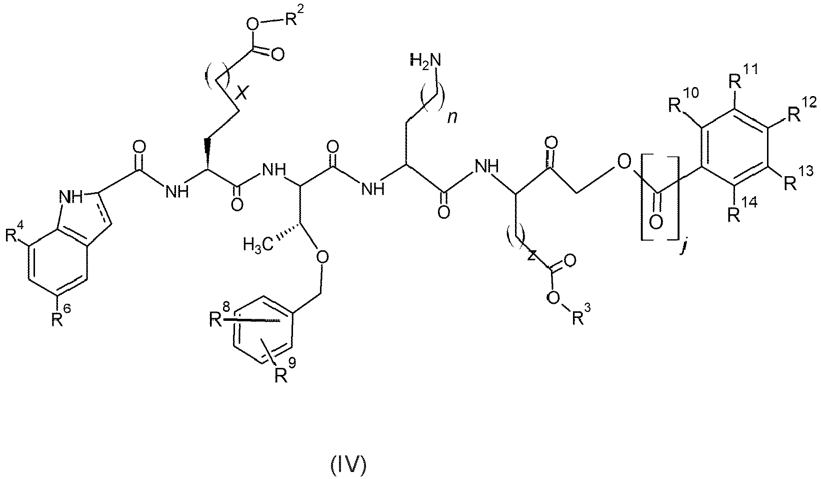 wherein 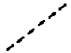
(the dotted line) in the heterocyclic ring of position P5 represents a bond which maybe present or absent, when present it forms a double bond in combination with the single bond already present;
R₂ and R₃ are independently chosen from hydrogen, or a C₁₋₄ alkyl including branched alkyl and cycloalkyl. Preferably R₂ and R₃ are independently chosen from a hydrogen, a methyl o an ethyl;
   R₄, and R₆ are each independently chosen from a hydrogen, a halogen or a C₁₋₄ alkyl including branched alkyl and cycloalkyl, wherein each C₁₋₄ alkyl is optionally substituted with 1 -3 halogen atoms. Preferably, R₄, and R₆ are each independently chosen from hydrogen, a halogen such as a fluorine or a trifluoromethyl;
   x, and z are each independently chosen from 0, 1, or 2 and n is 0, 1, 2 or 3. Preferably x, and z are 1 and n is 0, 1, 2 or 3;
   R₈ and R₉ are independently chosen from a hydrogen, a halogen or a C₁₋₄ alkyl including branched alkyl and cycloalkyl, wherein each C₁₋₄ alkyl is optionally substituted with 1 -3 halogen atoms. Preferably, R₈ and R₉ are independently chosen from a hydrogen or a halogen such as fluorine;
   j is 0 or 1; and
   R₁₀, R₁₁, R₁₂, R₁₃ and R₁₄ are each independently selected from a hydrogen, a halogen or a C1-4 alkyl including branched alkyl and cycloalkyl, phenyl, phenoxy, nitro, nitrile, PhCO, NHAc, wherein each alkyl is optionally substituted with 1 -3 halogen atoms. Preferably, R₁₄ or R₁₀ are selected from a halogen such as fluorine, a methyl or a trifluoromethyl, and R₁₁, R₁₂, and R₁₃ are each a hydrogen or a halogen such as fluorine.

In yet another embodiment, in the present invention we herein provide compounds of general formula (V), or a salt, solvate or stereoisomer thereof, 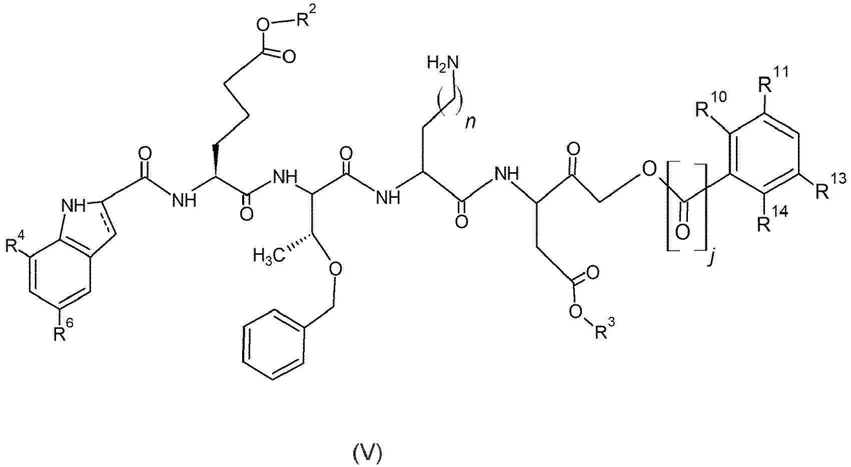 wherein 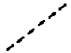
(the dotted line) in the heterocyclic ring of position P5 represents a bond which maybe present or absent, when present it forms a double bond in combination with the single bond already present;
R₂ and R₃ are independently chosen from a hydrogen or a methyl;
   R₄, and R₆ are each independently chosen from a hydrogen, or a halogen such as a fluorine;
   n is 0, 1, 2 or 3;
   j is 0 or 1; and
   R₁₄ or R₁₀ are selected from a halogen such as fluorine, or a methyl and R₁₁, and R₁₃ are each a hydrogen or a halogen such as fluorine;
   whereas when j is 0, then R₁₄, R₁₀ are a halogen such as fluorine, and R₁₁, and R₁₃ are each a halogen such as fluorine.

It is noted that all structures above cover all possible forms (eg one acid deprotonated, two acids deprotonated together with the amine being in both states), and position P5 may be present as drawn or may be the oxidise derivative wherein an additional double bond is present.

In a particular embodiment, the compound of formula (II) is selected from a compound (protonated or deprotonated) or a salt, solvate or stereoisomer thereof selected from the list consisting of:

| |
|---|
| NH-Idc-hGlu-Thr(Bzl)-Dab-Asp-AOMK |
| NH-Idc-hGlu-Thr(Bzl)-Dab-Asp(**O-Me**)-AOMK |
| NH-Idc-hGlu(**O-Me)**-Thr(Bzl)-Dab-Asp(**O-Me**)-AOMK |
| NH-Idc(**5-F**)-hGlu-Thr(Bzl)-Dab-Asp-AOMK |
| NH-Idc**(5-F)**-hGlu**(O-Me)**-Thr(Bzl)-Dab-Asp**(O-Me)**-AOMK |
| NH-Idc(**7-F**)-hGlu-Thr(Bzl)-Dab-Asp-AOMK |
| NH-Idc**(7-F)**-hGlu**(O-Me)**-Thr(Bzl)-Dab-Asp**(O-Me)**-AOMK |
| NH-Idc-hGlu-Thr(Bzl)-Dab-Asp-**O(2,3,S,6-F4-Ph)** |
| NH-Idc(**5-F**)-hGlu-Thr(Bzl)-Dab-Asp-**O(2,3,5,6-F4-Ph)** |
| NH-Idc(**5-F**)-hGlu(**O-Me**)-Thr(Bzl)-Dab-Asp(**O-Me**)-**O(2,3,5,6-F4-Ph)** |
| NH-Idc(**7-F**)-hGlu-Thr(Bzl)-Dab-Asp-**O(2,3,5,6-F4-Ph)** |
| NH-Idc(**7-F**)-hGlu(**O-Me**)-Thr(Bzl)-Dab-Asp(**O-Me**)-**O(2,3,S,6-F4-Ph)** |

In particular, the following chemical structures (protonated or deprotonated) or a salt, solvate or stereoisomer thereof selected from the any one of the following list, is particularly encompassed by the present invention:

| | |
|---|---|
| 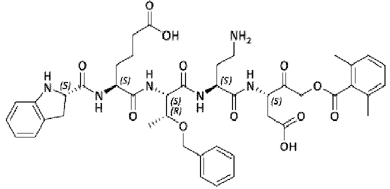 | 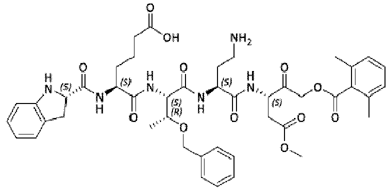 |
| **Cas 2_1: NH-Idc-hGlu-Thr(Bzl)-Dab-Asp-AOMK** | **Cas 2_2: NH-Idc-hGlu-Thr(Bzl)-Dab-Asp(O-Me)-AGMK** |
| 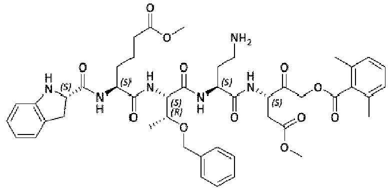 | 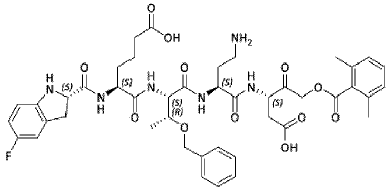 |
| **Cas 2_3: NH-Idc-hGlu(O-Me)-Thr(Bzl)-Dab-Asp(O-Me)-AOMK** | **Cas 2_4: NH-Idc(5-F)-hGlu-Thr(Bzl)-Dab-Asp-AOMK** |
| 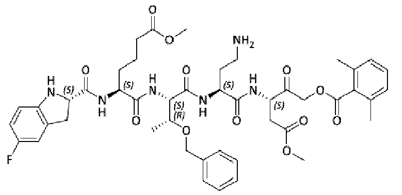 | 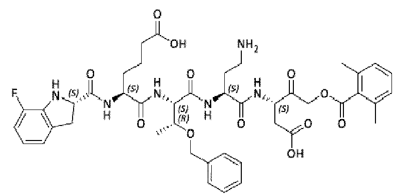 |
| **Cas 2_5: NH-Idc(5-F)-hGlu(O-Mel-Thr(Bzl)-Dab-Asp(O-Me)-AOMK** | **Cas 2_6: NH-Idc(7-F)-hGlu-Thr(Bzl)-Dab-Asp-AOMK** |
| 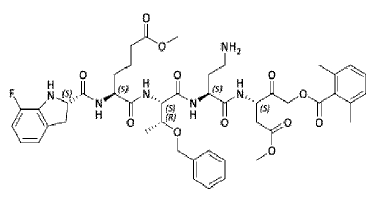 | 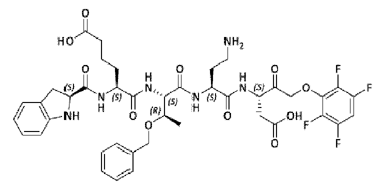 |
| **Cas 2_7: NH-Idc(7-F)-hGlu(O-Me)-Thr(Bzl)-Dab-Asp(O-Me)-AOMK** | **Cas 2_8: NH-Idc-hGlu-Thr(Bzl)-Dab-Asp-O(2,3,5,6-F4-Ph)** |
| 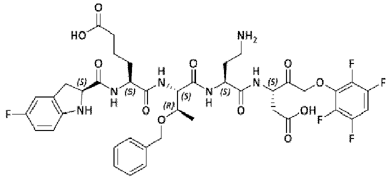 | 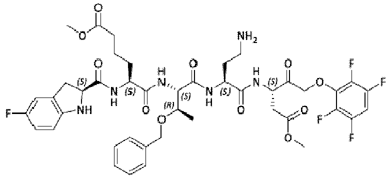 |
| **Cas 2_9: NH-Idc(5-F)-hGlu-Thr(Bzl)-Dab-Asp-O(2,3,5,6-F4-Ph) Cas** | **2_10: NH-Idc(5-F)-hGlu(O-Me)-Thr(Bzl)-Dab-Asp(O-Me)-O(2,3,5,6-F4-Ph)** |
| 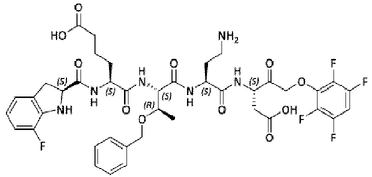 | 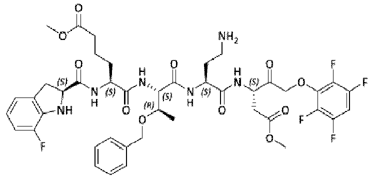 |
| **Cas 2_11: NH-Idc(7-F)-hGlu-Thr(Bzl)-Dab-Asp-O(2,3,5,6-F4-Ph)** | **Cas 2_12: NH-Idc(7-F)-hGlu(O-Me)-Thr(Bzl)-Dab-Asp(O-Me)-O(2,3,5,6-F4-Ph)** |

### Pharmaceutical compositions

Compounds of any of the above formulae of the invention where R1 is a warhead such as AOMK, are irreversible inhibitors of Caspase-2 and therefore can be used in the prevention or treatment of disorder or diseases mediates by this enzyme.

Accordingly, in a further aspect the present invention is directed to a pharmaceutical composition comprising a compound of any of the above formulae as defined herein wherein R₁ is a warhead such as AOMK, or a salt, solvate or stereoisomer thereof, and at least one pharmaceutically acceptable excipient.

The term "excipient" refers to a vehicle, diluent, or adjuvant that is administered with the active ingredient. Such pharmaceutical excipients can be sterile liquids, such as water and oils, including those of petroleum, animal, vegetable, or synthetic origin, such as peanut oil, soybean oil, mineral oil, sesame oil, and similar. Water or saline aqueous solutions and aqueous dextrose and glycerol solutions, particularly for injectable solutions, are preferably used as vehicles. Suitable pharmaceutical vehicles are described in "Remington's Pharmaceutical Sciences" by E.W. Martin, 21st Edition, 2005, or in "Handbook of Pharmaceutical Excipients," Rowe C.R.; Paul J.S.; Marian E.Q., sixth Edition, 2009.

The excipients and auxiliary substances necessary to manufacture the desired pharmaceutical form of administration of the pharmaceutical composition of the invention will depend, among other factors, on the elected administration pharmaceutical form. Said pharmaceutical forms of administration of the pharmaceutical composition will be manufactured according to conventional methods known by the skilled person in the art.

Examples of pharmaceutical compositions include any solid (tablets, pills, capsules, granules etc.) or liquid (solutions, suspensions or emulsions) compositions for oral, topical or parenteral administration.

In an embodiment the pharmaceutical compositions are in oral delivery form. Suitable dose forms for oral administration may be tablets and capsules and may contain conventional excipients known in the art such as binding agents, for example syrup, Arabic gam, gelatin, sorbitol, tragacanth, or polyvinylpyrrolidone; fillers, for example lactose, sugar, corn starch, calcium phosphate, sorbitol or glycine; lubricants for the preparation of tablets, for example magnesium stearate; disintegrants, for example starch, polyvinylpyrrolidone, sodium starch glycolate or microcrystalline cellulose; or pharmaceutically acceptable wetting agents such as sodium lauryl sulfate. Solid oral compositions can be prepared by conventional methods of blending, filling or tabletting. Such operations are conventional in the art. The tablets can be prepared, for example, by dry or wet granulation and optionally can be coated according to methods well known in normal pharmaceutical practice, in particular with an enteric coating.

Pharmaceutical compositions can also be adapted for parenteral administration, such as sterile solutions, suspensions or lyophilized products in the appropriate unit dosage form. Suitable excipients, such as bulking agents, buffering agents or surfactants, can be used.

The mentioned formulations can be prepared using standard methods such as those described or referred to in the European and US Pharmacopoeias and similar reference texts.

The compounds or compositions of the present invention may be administered by any suitable method, such as oral, sublingual, intranasal, intraocular, parenteral, subcutaneous, intramuscular, intravenous, or transdermal administration.

In general, the effective amount of a compound of the invention to be administered will depend on the relative efficacy of the compound chosen, the severity of the disorder being treated and/or prevented and the patient's weight. The active compounds can be administered one or more times a day, for example 1, 2, 3, or 4 times daily, with typical total daily doses in the range from about 0.01 mg/kg of body weight/day to about 1000 mg/kg of body weight/day. In another embodiment, the effective dosage amount of a compounds of the invention is about 500 mg/kg of body weight/day or less. In another embodiment, the effective dosage amount of a compound of the invention is about 100 mg/kg of body weight/day or less. In another embodiment, the effective dosage amount ranges from about 0.01 mg/kg of body weight/day to about 100 mg/kg of body weight/day of a compound of the invention; in another embodiment, from about 0.02 mg/kg of body weight/day to about 50 mg/kg of body weight/day; and in another embodiment, from about 0.025 mg/kg of body weight/day to about 20 mg/kg of body weight/day.

### Uses of the compounds of the invention

Compounds of the invention wherein R₁ is a group comprising a fluorophore moiety can be used as activity-based probes to determine Caspase-2 activity.

Accordingly, in another aspect the invention refers to the *in vitro* use of a compound of the invention as defined herein wherein R₁ is a group comprising a fluorophore moiety, or a salt, solvate or stereoisomer thereof, to determine Caspase-2 activity.

In a particular embodiment, Caspase-2 activity in a cell or a tissue can be determined by contacting a compound as defined herein wherein R₁ is a group comprising a fluorophore moiety, or a salt, solvate or stereoisomer thereof, with a sample comprising said cell or tissue and measuring the fluorescent signal emitted upon light excitation.

Compounds of any of the above formulae of the invention where R1 is a warhead such as AOMK comprise groups that react with the active site in the target enzyme (Caspase-2), thereby providing irreversible inhibition effects. Therefore, compounds of the invention wherein R₁ is a group can be used in the prevention or treatment of diseases or disorders in which Caspase-2 activity is involved.

Accordingly, in another aspect, the invention is directed to a compound of the invention wherein R₁ is a warhead such as AOMK, or a salt, solvate or stereoisomer thereof, for use as a medicament.

In another aspect, the invention is directed to a compound of the invention where R1 is a warhead such as AOMK, or a salt, solvate or stereoisomer thereof, for use in the prevention and/or treatment of a disease or disorder associated with an increased Caspase-2 activation selected from degenerative diseases such as Alzheimer's disease, Huntington's disease, Parkinson's disease, mild cognitive impairment, amyotrophic lateral sclerosis and Creutzfeld-Jacob disease, adrenoleukodystrophy; neonatal brain damage in particular neonatal brain ischemia; traumatic brain injury; kidney ischemia; hypoxia-ischemia injuries; stroke-like situations brain injuries; heart ischemia; myocardial infarction; amyotrophic lateral sclerosis; retinal damages; ophthalmic diseases such as age-macular degeneration, diabetic retinopathy, retinitis pigmentosa, blunt ocular injury, ischemic optic neuropathy and glaucoma; cytotoxicity prevention, particularly cytotoxicity mediated by chemicals, by physical agents such as radiation and acoustic trauma; skin damages; sterile inflammatory diseases such as diabetes, atherosclerosis, gout, pseudogout, joint loosening, atherosclerosis, syndromes triggered by aluminium salts, non-arteritic ischemic optic neuropathy, glaucoma and metabolic diseases; non-sterile inflammatory diseases such as bacterial infection in particular with bacteria producing pore-forming toxins, influenza virus infection and single-stranded RNA Rhabdoviridae infection, such as Maraba virus or vesicular stomatitis virus; diseases caused by pathogenic bacteria, such as Brucella, Staphylococcus aureus and Salmonella; dyslipidemias; obesity; metabolic syndrome; and nonalcoholic fatty liver disease, such as nonalcoholic steatohepatitis (NASH) and nonalcoholic fatty liver disease (NAFLD).

In a preferred embodiment, the invention is directed to a compound of the invention where R1 is a warhead such as AOMK, or a salt, solvate or stereoisomer thereof, for use in the prevention and/or treatment of nonalcoholic fatty liver disease (NAFLD).

The term "treatment" or "to treat" in the context of this specification means administration of a compound or composition according to the invention to ameliorate or eliminate the disease or one or more symptoms associated with said disease. "Treatment" also encompasses ameliorating or eliminating the physiological sequelae of the disease.

The term "ameliorate" in the context of this invention is understood as meaning any improvement on the situation of the patient treated.

The term "prevention" or "to prevent" in the context of this specification means administration of a compound or composition according to the invention to reduce the risk of acquiring or developing the disease or one or more symptoms associated with said disease.

In an embodiment, any method or use described herein can further comprise administering to the patient at least one other therapeutic agent.

The present invention is additionally explained below by means of examples. This explanation must by no means be interpreted as a limitation of the scope of the invention as it is defined in the claims.

### EXAMPLES

### BIOLOGICAL ASSAYS

### Example 1. Enzymatic kinetic studies with substrates

### Materials and methods

### Reagents

Fmoc-protected amino acids were purchased from Iris Biotech GmbH (Marktredwitz, Germany), Sigma-Aldrich (Poznan, Poland), Bachem (Torrance, CA, USA), Creosalus (Louisville, KY, USA), PE Biosciences Limited (Hong Kong, China) and Combi-Blocks (San Diego, USA). Fmoc-ACC fluorescent dye was synthesized according to the procedure published previously by Maly et al. Rink Amide AM resin (200-300 mesh, loading 0.48mmol/g), (2-(1H-benzotriazol-1-yl)-1,1,3,3-tetra-methyluronium hexafluorophosphate (HBTU), 1-[Bis(dime- thylamino) methylene]-1H-1,2,3-triazolo[4,5-b]pyridinium 3-oxid hexafluorophosphate (HATU), piperidine (PIP), diisopropylcarbodiimide (DICI), and trifluoroacetic acid (TFA) were purchased from Iris Biotech GmbH. Anhydrous HOBt was purchased from Creosalus. 2,4,6-trimethylpyridine (2,4,6-collidine), acetonitrile (ACN, HPLC gradient grade), triisopropylsilane (TIPS), were purchased from Sigma-Aldrich. N,N '-dimethylformamide (DMF, pure for analysis), methanol (MeOH), dichloromethane (DCM), AcOH, diethyl ether (Et2O), and phosphorus pentoxide (P2O5) were from POCh (Gliwice, Poland). All ACC-labeled fluorescent substrates were purified by reverse phase HPLC on Waters system (Waters M600 solvent delivery module and Waters M2489 detector system) using semi-preparative Discovery^{®} C8 column (particle size 10 µm). The solvent composition for substrates purification and LC-MS analysis was as follows: phase A (water/0.1% TFA), phase B (ACN/0.1% TFA). For the purification, the assay was run for 30 min in a linear gradient (from 95% phase A to 5% phase A). The purity and molecular mass [m/z+H]⁺ were determined using LC-MS Waters instrument using analytical Discovery^{®} C8 column (particle size 10 µm). The LC-MS assay was run for 20 min from 95% phase A to 5% phase A. All the compounds were at least 95% pure.

### Synthesis of substrates

Substrates with the general formula of NH-ldc-hGlu-Thr(Bzl)-P2-Asp-ACC were synthesized on a solid support according to the general methods described previously. All the amino acids have *L* configuration, unless otherwise stated.
For all the synthesized substrates, around 10 g (1 eq., 4.8 mmol) of Fmoc-protected Rink Amide resin (0.48 mol/g) was placed into 250mL glass cartridge for solid-phase synthesis and swollen in DCM for 30 min. Then the DCM was drained, and the resin was washed three times with DMF. Next, the Fmoc- protecting group was removed using 20% piperidine in DMF in three cycles (5 min, 5 min, and 25 min), and the resin was washed six times with DMF. About 2.5 eq. of Fmoc-ACC-OH (12 mmol, 5.3 g) was pre-activated with 2.5 eq. of HOBt (12 mmol, 1.8 g) and 2.5 eq. of DICI (12 mmol, 1.6 mL) in a minimal amount of DMF for 3 min and poured onto the resin. The cartridge was gently agitated for 30 min, and more of the DMF was added as the mixture became too dense. The reaction was carried out for 24 h, followed by filtration and washing the resin three times with DMF. The Fmoc-ACC-OH coupling was repeated to improve the coupling yield using 1.5 eq. of the above reagents. After 24 h, the resin was washed three times with DMF, and the ninhydrin test was performed to confirm the complete Fmoc-ACC-OH coupling. Then the Fmoc group was removed from ACC by using 20% piperidine in DMF, followed by washing the resin six times with DMF. Next, 2.5 eq. of Fmoc-Asp(tBu)-OH (12 mmol, 5.3 g) was pre-incubated with 2.5 eq. of HATU (12 mmol, 4.6 g) and 2.5 eq. of 2,4,6-collidine (12 mmol, 1.6 mL) in DMF for 3 min and poured onto the H2N-ACC-resin. The reaction was carried out for 24 h and repeated for another 24 h with the use of 1.5 eq. of the Fmoc-Asp(tBu)-OH/HATU/2,4,6-collidine reagents. Then the Fmoc-Asp(tBu)-ACC-resin was washed three times with DMF, followed by Fmoc de-protection with 20% piperidine in DMF, and washing the resin six times with DMF, three times with DCM, three times with MeOH and dried in a desiccator over P₂O₅ overnight. The obtained resin (around 12g) was divided into 100mg portions and used for the synthesis of individual caspase-2 substrates.

### P2 substrates synthesis

### ACC-labeled fluorescent substrates with the general formula of NH-ldc-hGlu-Thr(Bzl)-

P2-Asp-ACC were synthesized using a MultiChem 48-well synthesis apparatus (FlexChem from SciGene, CA, USA). For each of the substrates, 100mg each, 0.05 mmol of NH₂-Asp(tBu)-ACC-resin were placed in separated wells of multi-well cartridge and DCM was added to swell the resin. Next, the DCM was filtered out and the resin was washed three times with DMF. In thirty 1.5mL tubes, 3eq. of various amino acids (0.15 mmol) was mixed with 1mL of DMF containing 3eq. of HATU (0.15mmol, 60mg) and 3eq. of 2,4,6-collidine (0.15mmol, 20mL) and poured onto the resin. The P2 coupling reaction was carried out for 3 hours, followed by ninhydrin test. Fmoc group was then removed from each substrate with 20% piperidine in DMF, and the resin in each well was washed six times with DMF. Next, 3eq. (x30) of Fmoc-Thr(Bzl)-OH (4.5mmol, 1.95g) was preincubated with 3eq. (x30) of HATU (4.5mmol, 1.72g) and 2,4,6-collidine (4.5mmol, 600uL) in a minimal amount of DMF for 1 min and poured onto the resin. The P3 coupling reaction was carried out for 3 hours, followed by ninhydrin test. Fmoc group was then removed from each substrate with 20% piperidine in DMF, and the resin in each well was washed six times with DMF. Next, 3 eq. (x30) of Fmoc-hGlu(tBu)-OH (4.5 mmol, 2.0g) was preincubated with 3eq. (x30) of HATU (4.5mmol, 1.72g) and 2,4,6-collidine (4.5mmol, 600mL) in a minimal amount of DMF for 1 min and poured onto the resin. The P4 coupling reaction was carried out for 3 hours, followed by ninhydrin test. Fmoc group was then removed from each substrate with 20% piperidine in DMF, and the resin in each well was washed six times with DMF. Next, 3eq. (x30) of Fmoc-Idc-OH (4.5mmol, 1.75g) was preincubated with 3eq. (x30) of HATU (4.5mmol, 1.72g) and 2,4,6-collidine (4.5mmol, 600mL) in a minimal amount of DMF for 1 min and poured onto the resin. The P5 coupling reaction was carried out for 3 hours, followed by ninhydrin test. Fmoc group was then removed from each substrate with 20% piperidine in DMF, and the resin in each well was washed six times with DMF, three times with DCM, three times with MeOH and dried in a desiccator over P₂O₅ overnight. All substrates were cleaved from the resin using ice-cold TFA/TPS/water (% v/v/v, 95/2.5/2.5) mixture for 2 h (shaking once per 15 min). The solution from each well was collected separately and the remaining resin was washed with TFA. Substrates were then precipitated with ice-cold Et₂O for 30min and centrifuged. Next, the supernatant was discarded, and the pellet was re-suspended in ice-cold Et₂O and centrifuged again. The supernatant was then discarded, and the pellet was allowed to dry out, and the crude produce was dissolved in 1mL of DMSO and purified on HPLC. The pure substrate was collected, frozen at -80°C and lyophilized. The final product (white powder) was then dissolved in dimethyl sulfoxide (DMSO) to the final concentration of 20 mM and stored at -80°C until use.

### Preparation of recombinant caspases

The detailed protocol for the expression and purification of human apoptotic caspases can be found elsewhere (Stennicke, 1999).

### Enzymatic kinetic studies

The screening of P2 substrates towards caspase -2, -3, and -8 was performed using a fMax spectrofluorimeter (Molecular Devices, Sunnyvale, CA, USA) operating in fluorescence kinetic mode in 96-well Corning (Corning, NY, USA) plates. The ACC fluorescence was monitored using 355 nm (excitation) and 460 nm (emission) wavelengths. Before kinetic analysis, all caspases were active site-titrated using the zVAD-fmk inhibitor (Cayman Chemical Company, Cat No. 14467). The caspases assay buffer was 10% w/v sucrose, 1M sodium citrate, 20 mM Pipes, 10 mM NaCl, 1 mM EDTA, and 10 mM DTT (pH = 7.3). Buffers were prepared at room temperature, and all kinetic assays were performed at 37°C. All enzymes were preincubated for 15min before any activity assays. Substrates were screened at the concentration of 10mM, and the caspases concentrations were as follow: caspase-2 5nM, caspase-3 15nM, and caspase-8 40nM. The total volume in a single well was 100mL. The total assay time was 30 min, but only the linear portion of the fluorescence progress curve was taken for the analysis. The kinetic data were analyzed using Graph Pad Prism software. Data are presented as the rate of fluorescence liberation/production (in RFU/s relative fluorescence units per second) per 10nM of caspase.

### Results

We aimed to develop highly specific and selective caspase-2 substrates by revisiting its catalytic preferences at the P2 (also referred to throughout the present invention as AA) position. According to what is generally accepted, "specific" means high kinetic parameters (for example high kcat/KM for substrats, or high kobs/I for inhibitors) and "selective" means that some reagents (substrates/inhibitors) target only one enzyme. The main objective of this part of the invention was to synthesize and biochemically evaluate a series of pentapeptide fluorescent substrates that were designed based on the NH-ldc-hGlu-Thr(Bzl)-**Ser**-Asp-ACC (WRMP23) formula, which was previously published as the most selective caspase-2 substrate (Poreba et al., Cell Death & Differentiation 2019, 26, 2695-2709). From the above mentioned WRMP23 formula, the following scaffold (see scaffold 1 below) was designed, which forms the bases for the newly develop highly specific and selective caspase-2 substrates and inhibitors of the present invention:

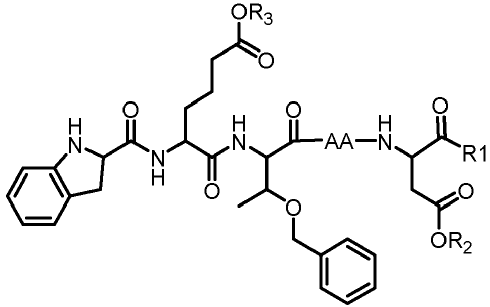

Scaffold 1. NH-Idc-hGlu-Thr(Bzl)-**AA**-Asp-R₁. R1: -ACC (substrates) or -AOMK (inhibitors). R3: H. R2: H. AA: P2.

Results are presented herein as a rate of substrate hydrolysis expressed as RFU/s, where RFU is Relative Fluorescence Unit per 10nM of caspase (see figure 1 and table 1).

**Table 1.**

| | **RFU/s/10nM** | | | | |
|---|---|---|---|---|---|
| **Sequence: NH-ldc-hGlu-Thr(Bn)-AA-Asp-ACC** | **Caspase-2** | **Caspase-3** | **Caspase-8** | **Casp-2 vs Casp-3** | **Casp-2 vs Casp-8** |
| Ser | 182,81 | 4,75 | 6,57 | 38,49 | 27,82 |
| Thr | 138,64 | 91,18 | 24,99 | 1,52 | 5,55 |
| VaI | 60,94 | 315,52 | 12,81 | 0,19 | 4,76 |
| Ala | 69,13 | 25,77 | 7,50 | 2,68 | 9,22 |
| His | 366,05 | 66,18 | 31,04 | 5,53 | 11,79 |
| Ile | 63,92 | 423,23 | 20,11 | 0,15 | 3,18 |
| Arg | 97,18 | 6,25 | 2,32 | 15,55 | 41,89 |
| Lys | 90,58 | 0,48 | 1,64 | 188,71 | 55,23 |
| His(Bzl) | 181,98 | 95,02 | 35,75 | 1,92 | 5,09 |
| Ser(Bzl) | 50,41 | 208,69 | 26,13 | 0,24 | 1,93 |
| Cys(Bzl) | 18,89 | 94,66 | 14,74 | 0,20 | 1,28 |
| hPhe | 45,50 | 31,88 | 23,26 | 1,43 | 1,96 |
| Aoc | 19,94 | 102,85 | 28,29 | 0,19 | 0,70 |
| hLeu | 24,48 | 64,17 | 10,06 | 0,38 | 2,43 |
| Nva | 55,38 | 166,48 | 7,39 | 0,33 | 7,49 |
| Chg | 3,39 | 118,07 | 3,30 | 0,03 | 1,03 |
| Cit | 34,24 | 18,45 | 4,86 | 1,86 | 7,05 |
| Phe(2F) | 12,56 | 34,76 | 4,65 | 0,36 | 2,70 |
| Phe(4F) | 17,64 | 38,76 | 3,98 | 0,46 | 4,43 |
| Phe(4I) | 11,21 | 43,12 | 5,12 | 0,26 | 2,19 |
| Hnv | 25,43 | 15,98 | 1,15 | 1,59 | 22,11 |
| Tie | 8,54 | 7,65 | 2,23 | 1,12 | 3,83 |
| Cha | 18,44 | 8,51 | 3,12 | 2,17 | 5,91 |
| Phe(guan) | 21,21 | 49,23 | 6,08 | 0,43 | 3,49 |
| hTyr | 16,77 | 23,94 | 9,56 | 0,70 | 1,75 |
| 3-Pal | 8,43 | 11,29 | 1,83 | 0,75 | 4,61 |
| Met(O2) | 5,43 | 4,97 | 0,55 | 1,09 | 9,87 |
| Phe(4NH2) | 17,21 | 39,65 | 4,28 | 0,43 | 4,02 |
| beta-Ala | 4,32 | 6,18 | 0,65 | 0,70 | 6,65 |

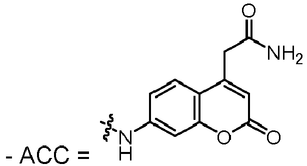

As shown in the tables above, the inventors of the present invention have found that compounds of formula below: 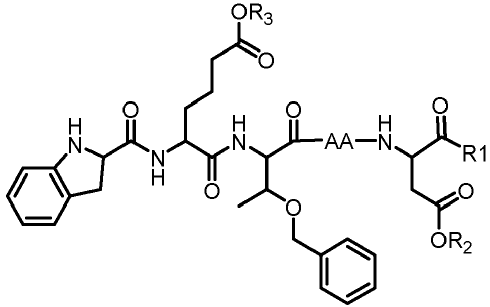 with a Lys in position AA instead of Serine, present a high Caspase-2 activity and remarkable selectivity over Caspase-3 or Caspase-8, thus leading to improved Caspase-2 substrates. The inventors have further found that compounds of the above formula with a His in position AA instead of Serine present a strong Caspase-2 activity.

### Example 2. Enzymatic kinetic studies with inhibitors

### Materials and methods

### Reagents

Fmoc-protected amino acids were purchased from Iris Biotech GmbH (Marktredwitz, Germany), Sigma-Aldrich (Poznan, Poland), Bachem (Torrance, CA, USA), Creosalus (Louisville, KY, USA), PE Biosciences Limited (Hong Kong, China) and Combi-Blocks (San Diego, USA). 2-chlorotrityl chloride resin (100-200 mesh, loading 1.59 mmol/g), 1-[Bis(dime-thylamino) methylene]-1H-1,2,3-triazolo[4,5-b]pyridinium 3-oxid hexafluorophosphate (HATU), piperidine (PIP), diisopropylcarbodiimide (DICI), and trifluoroacetic acid (TFA) were purchased from Iris Biotech GmbH. 2,4,6-trimethylpyridine (2,4,6-collidine), acetonitrile (ACN, HPLC gradient grade), triisopropylsilane (TIPS), hydrobromic acid solution (30% HBr wt. in acetic acid (AcOH)), N-methylmorpholine (NMM), tetrahydrofuran (THF anhydrous), isobutylchloroformate (IBCF), and 2,6-dimethylbenzoic acid (2,6-DMBA) were purchased from Sigma-Aldrich. N,N '-dimethylformamide (DMF, pure for analysis), methanol (MeOH), dichloromethane (DCM), AcOH, diethyl ether (Et2O), and phosphorus pentoxide (P2O5) were from POCh (Gliwice, Poland). Diazomethane for the acyloxymethyl ketone (AOMK) inhibitor synthesis was generated according to the Aldrich Technical Bulletin (AL-180) protocol. All AOMK-inhibitors were purified by reverse phase HPLC on Waters system (Waters M600 solvent delivery module and Waters M2489 detector system) using semi-preparative Discovery^{®} C8 column (particle size 10 µm). The solvent composition for inhibitors purification and LC-MS analysis was as follows: phase A (water/0.1% TFA), phase B (ACN/0.1% TFA). For the purification, the assay was run for 30 min in a linear gradient (from 95% phase A to 5% phase A). The purity and molecular mass [m/z+H]⁺ were determined using LC-MS Waters instrument using analytical Discovery^{®} C8 column (particle size 10 µm). The LC-MS assay was run for 20 min from 95% phase A to 5% phase A. All the compounds were at least 95% pure.

### P2 inhibitors synthesis

The detailed kinetic analysis of fluorogenic substrates for apoptotic caspases in P2 positions allowed us to select caspase-2 selective tetrapeptide motifs and use them to design irreversible AOMK-tagged inhibitors. Inhibitors with the general formula of NH-Idc-hGlu-Thr(Bzl)-AA-Asp-AOMK were synthesized according to the general methods described previously (Poreba 2019, Poreba 2016). As a control inhibitor, Ac-VDVAD-AOMK was synthesized. The detailed procedure for the AOMK-based inhibitor synthesis is exemplified with the synthesis of NH-ldc-hGlu-Thr(Bzl)-Ser-Asp-AOMK (NH-23-C2, Poreba et al., Cell Death & Differentiation 2019, 26, 2695-2709). All other inhibitors were synthesized and purified in the same manner. All the amino acids have L configuration, unless otherwise stated.

**Synthesis of NH-ldc-hGlu-Thr(Bzl)-Ser-Asp-AOMK (NH-23-C2).** A 0.2 M solution of the Boc-Asp(tBu)-OH amino acid (5 mmol, 1.45g) in anhydrous THF was stirred in an ice/acetone bath at -10°C for 10 min. Then, 4-methylmorpholine (6.25 mmol, 1.25 eq.) and isobutyl chloroformate (5.75 mmol, 1.15 eq.) were added. The reaction was carried out at -10°C for 45min. In a parallel experiment, diazomethane was generated according to the Aldrich Technical Bulletin (AL-180) protocol. Next, the solution of mixed anhydrides was added dropwise to the ethereal diazomethane (16.6-21.4 mmol) at 0°C. The mixture was stirred for 10 min, and after this time the ice bath was removed and the reaction was carried out for 2h at room temperature. To obtain Boc-Asp(tBu)-CH₂Br, 15 mL of a 1:2 solution of HBr (30% wt. in CH₃COOH) and water was added dropwise to the mixture over a period of 10 min. Immediately after, the mixture was diluted with ethyl acetate, transferred to a separatory funnel and extracted with water (once), saturated aqueous NaHCOs (twice) and brine (twice). The organic fraction was dried over MgSO₄ and evaporated under reduced pressure. The product was obtained as a pale yellow oil and used for the synthesis without further purification. The product purity determined by HPLC was > 95%, and the overall yield was > 90%. In the next reaction, 1 eq of Boc-Asp(tBu)-CH₂Br was dissolved in a small volume of DMF, followed by the addition of KF (3 eq.) and 2,6-dimethylbenozic acid (2,6-DMBA) (1.2 eq.). The mixture was stirred for 25min in inert atmosphere of argon. After the reaction was completed (HPLC analysis) the solution was diluted with ethyl acetate, transferred to a separatory funnel, and extracted with 5% of citric acid (twice), 5% of aqueous NaHCO₃ (twice) and brine (twice). Next, the organic fraction was dried over MgSO₄ and evaporated under reduced pressure. The product was obtained as a yellow oil (yield >95%) and used for probes synthesis without further purification. Boc-Asp(tBu)-AOMK (100 mg/0.213 mmol) was added to a solution of 25% TFA in DCM. The Boc- and tBu- groups deprotection was carried out for 30 min. Then TFA and DCM were evaporated under reduced pressure, and the final product (NH₂-Asp-AOMK) was obtained as a yellow oil and used without further purification (yield 98% by HPLC) (**Block B,** Synthetic scheme 1). In a separate synthesis the NH-ldc-hGlu(tBu)-Thr(Bzl)-Ser(tBu)-OH peptide fragment was synthesized using 2-chlorotrityl chloride resin (200mg, 1.6mmol/g, 0.33mmol). Fmoc-*L*-Ser(tBu)-OH (3eq, 1mmol, 383mg) was dissolved in the minimal volume of anhydrous DCM, before 4.5eq of DIPEA (1.5mmol, 260uL) was added and the mixture activated for 1 min and poured onto the resin. The reaction mixture was agitated for 3h. Then the Fmoc group was removed with 20% piperidine in DMF and the resin was washed six times with DMF. Then, Fmoc-Thr(Bzl)-OH (3eq., 1mmol, 431mg) and HATU (3eq., 1mmol, 380mg) were dissolved in a minimal volume of DMF and 3eq of 2,4,6-collidine (1mmol, 130uL) was added. The mixture was poured onto resin and the reaction was carried out for 3 hours. After this time, Fmoc group was removed with 20% piperidine in DMF and the resin was washed six times with DMF. Next, Fmoc-hGlu(tBu)-OH (3eq., 1mmol, 440mg) and HATU (3eq., 1mmol, 380mg) were dissolved in a minimal volume of DMF and 3eq of 2,4,6-collidine (1mmol, 130uL) was added. The mixture was poured onto resin and the reaction was carried out for 3 hours. After this time, Fmoc group was removed with 20% piperidine in DMF and the resin was washed six times with DMF. Next, Fmoc-Idc-OH (3eq., 1mmol, 386mg) and HATU (3eq., 1mmol, 380mg) were dissolved in a minimal volume of DMF and 3eq of 2,4,6-collidine (1mmol, 130uL) was added. The mixture was poured onto resin and the reaction was carried out for 3 hours. After this time, Fmoc group was removed with 20% piperidine in DMF and the resin was washed six times with DMF, three times with DCM and three times with MeOH. The resin was then dried in a desiccator over P2O5 overnight. Next the peptide was cleaved from the resin during 45 min incubation in a mixture of DCM/TFE/AcOH (v/v/v, 8:1:1). The solution was then filtered, and solvents were removed under reduced pressure. The crude peptide was dissolved in acetonitrile:H₂O (v/v, 7:3) and lyophilized to obtain NH-ldc-hGlu(tBu)-Thr(Bzl)-Ser(tBu)-OH as a white powder (**Block A,** Synthetic scheme 1). The peptide purity was > 95% and used for the NH-23-C2 inhibitor synthesis without further purification. Next, the peptide fragment (1eq.) was coupled with NH2-Asp-AOMK (1eq.) in DMF and using HATU (1eq.) and 2,4,6-collidine (5eq.) as coupling reagents. The reaction was carried out for 2 hours at room temperature, and after this time the mixture was injected into HPLC, and NH-ldc-hGlu(tBu)-Thr(Bzl)-Ser(tBu)-Asp-AOMK was purified and lyophilized. The product was then dissolved in a mixture of DCM:TFA 1:2 to remove the protecting group. The reaction was carried out for 1 hour at room temperature, and the DCM:TFA mixture was removed with argon. The crude product was purified on HPLC and lyophilized to yield final compound: NH-ldc-hGlu-Thr(Bzl)-Ser-Asp-AOMK (**Block C,** Synthetic scheme 1). The molecular mass [m/z+H]⁺ and purity were confirmed by LC-MS analysis. In the similar manner other inhibitors were synthesized. Also, the reference inhibitor Ac-VDVAD-AOMK (Ac-Val-Asp-Val-Ala-Asp-AOMK) was synthesized with this procedure, however, in this case the peptide fragment was additionally N-capped with acetyl group. Acetic acid (5eq.) and HBTU (5eq.) were dissolved in a minimal amount of DMF and poured onto NH2-Val-Asp(tBu)-Val-Ala-resin (1eq) and the acetylation reaction was carried out for 30 min. N-acetylated peptide was then proceeded with the procedure described above.

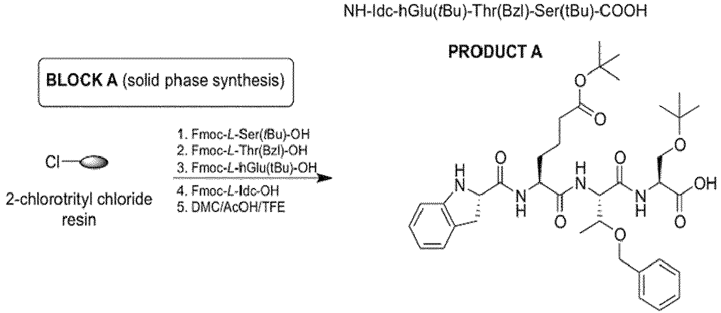

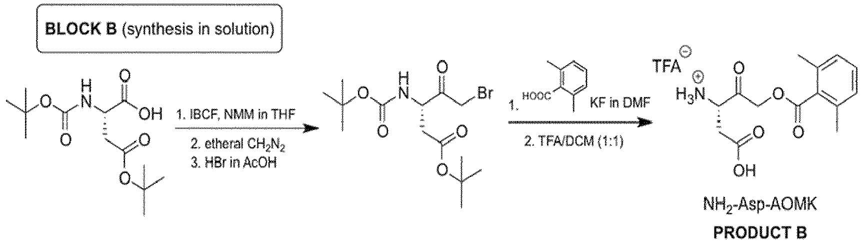

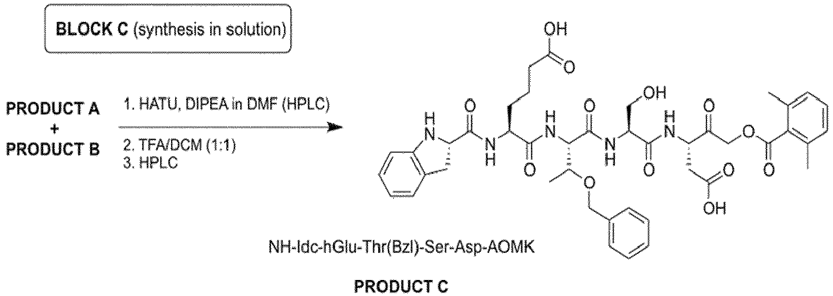

### Synthetic scheme 1. AOMK-based inhibitor synthesis procedure exemplified by the synthesis of NH-Idc-hGlu-Thr(Bzl)-Ser-Asp-AOMK

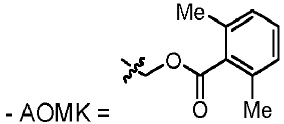

**Table 2. Structures of inhibitors with general formula NH-ldc-hGlu-Thr(Bzl)-AA-Asp-AOMK.**

| | |
|---|---|
| 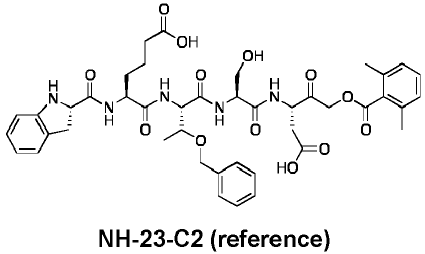 | 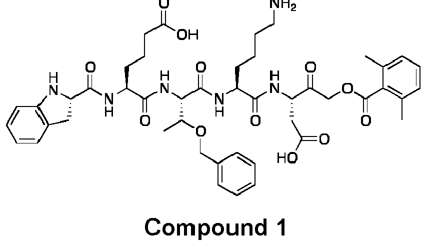 |
| NH-Idc-hGlu-Thr(Bzl)-Ser-Asp-AOMK | NH-Idc-hGlu-Thr(Bzl)-**Lys**-Asp-AOMK |
| 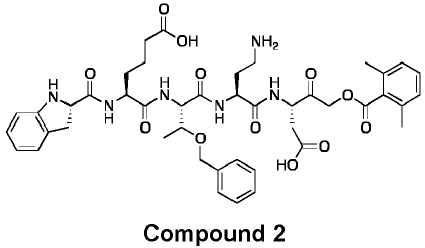 | 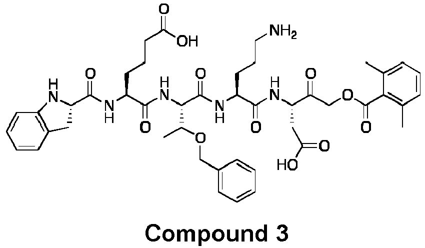 |
| NH-Idc-hGlu-Thr(Bzl)-**Dab**-Asp-AOMK | NH-Idc-hGlu-Thr(Bzl)-**Orn**-Asp-AOMK |
| 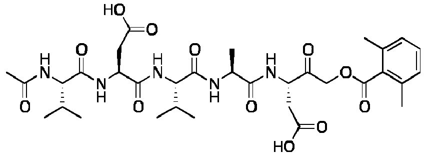 | |
| **AC-VDVAD-AOMK** Ac-Val-Asp-Val-Ala-Asp-AOMK | |

For the synthesis of NH-ldc-hGlu-Thr(Bzl)-Lys-Asp-AOMK, Fmoc-L-Lys(Boc)-OH was used instead Fmoc-L-Ser(tBu)-OH. For the synthesis of NH-ldc-hGlu-Thr(Bzl)-Orn-Asp-AOMK, Fmoc-L-Orn(Boc)-OH was used instead Fmoc-L-Ser(tBu)-OH. For the synthesis of NH-ldc-hGlu-Thr(Bzl)-Dab-Asp-AOMK, Fmoc-L-Dab(Boc)-OH was used instead Fmoc-L-Ser(tBu)-OH. For the synthesis of NH-ldc-hGlu-Thr(Bzl)-Dap-Asp-AOMK, Fmoc-L-Dab(Boc)-OH was used instead Fmoc-L-Ser(tBu)-OH. For the synthesis of NH-Idc-hGlu-Thr(Bzl)-Arg-Asp-AOMK, Fmoc-L-Arg(Pbf)-OH was used instead Fmoc-L-Ser(tBu)-OH.

### Enzymatic kinetic studies - determination of kobs/l inhibition parameter

The measurement of k_{obs}/l inhibition parameter (second order rate of enzyme inhibition) of synthesized inhibitors towards caspases-2, -3, and -8 was performed using a fMax spectrofluorimeter (Molecular Devices, Sunnyvale, CA, USA) operating in fluorescence kinetic mode in 96-well Corning (Corning, NY, USA) plates. Before kinetic analysis, all caspases were active site-titrated using the zVAD-fmk inhibitor (Cayman Chemical Company, Cat No. 14467). The caspases assay buffer was 10% w/v sucrose, 1M sodium citrate, 20 mM Pipes, 10 mM NaCl, 1 mM EDTA, and 10 mM DTT (pH = 7.3). Buffers were prepared at room temperature, and all kinetic assays were performed at 37°C. The k_{obs}/l parameter was measured under pseudo-first-order kinetic conditions ([I] » [E]). Inhibitor (20uL) was diluted in a 96-well plate and mixed with the appropriate substrate (20uL); NH-ldc-hGlu-Thr(Bzl)-Ser-Asp-ACC (50uM) for caspase-2, Ac-DEVD-ACC (100uM) for caspase-3 and Ac-LEHD-ACC (100uM) for caspase-8. The ACC fluorescence was monitored using 355 nm (excitation) and 460 nm (emission) wavelengths. The substrate-inhibitor mixture (40uL in total) were pre-incubated at 37C for 15 min. At the same time in a separate tube, the caspase was preincubated in assay buffer at 37C, and after 15 min, the enzyme was added to wells (60uL per well, total reaction volume 100uL) and the fluorescence was monitored for 30min, starting immediately. Second-order rate of inhibition (k_{obs}/l) was determined in at least three independent experiments and presented as an average value. GraphPad Prism 7 software was used for calculations (see figure 2).

### Preparation of recombinant caspases.

The detailed protocol for the expression and purification of human apoptotic caspases can be found elsewhere (Stennicke, 1999).

### Enzymatic kinetic studies - determination of Kᵢ and IC₅₀ inhibition parameters

The measurement of Kᵢ and IC₅₀ inhibition parameter of synthesized inhibitors towards caspases-2, -3, and -8 was performed using a fMax spectrofluorimeter (Molecular Devices, Sunnyvale, CA, USA) operating in fluorescence kinetic mode in 96-well Corning (Corning, NY, USA) plates. Before kinetic analysis, all caspases were active site-titrated using the zVAD-fmk inhibitor (Cayman Chemical Company, Cat No. 14467). The caspases assay buffer was 10% w/v sucrose, 1M sodium citrate, 20 mM Pipes, 10 mM NaCl, 1 mM EDTA, and 10 mM DTT (pH = 7.3). Buffers were prepared at room temperature, and all kinetic assays were performed at 37°C. The Kᵢ parameter was measured using the Morrison equation (Copeland, 2000). For each caspase, an appropriate ACC-fluorogenic substrate was used to monitor substrate hydrolysis: NH-ldc-hGlu-Thr(Bzl)-Ser-Asp-ACC (50uM) for caspase-2, Ac-DEVD-ACC (100uM) for caspase-3 and Ac-LEHD-ACC (100uM) for caspase-8. The ACC fluorescence was monitored using 355 nm (excitation) and 460 nm (emission) wavelengths. To meet the criteria for reversible inhibitor kinetics, the lowest inhibitor concentration was at least 4-fold higher that the caspase concentration in the assay. The caspase (60uL) was first pre-warmed in the assay buffer in the 96-well plate for 15 min at 37C and then pre-incubated with the inhibitor (20uL) for another 15 min at 37C. Next, the substrate (20uL) was added to the enzyme-inhibitor mixture and the progress of reaction (ACC release) was monitored overtime. The Kᵢ parameter was calculated from Morrison equation, and the IC₅₀ parameter was calculated using the formula: IC₅₀ = Kᵢ × (1+[S]/Kₘ], where the [S] is substrate concentration used in the assay and Km is a Michaelis-Menten constant for a substrate. All the measurements were performed at least three times and the data was analyzed using GraphPad Prism 7 software.

Data for Ki and IC50 (for caspase-2) is shown in figure 3.

Results are presented as kobs/l, Ki and IC50 values are indicated in tables 3 to 5 below.

**Table 3**

| | Caspase-2 | | |
|---|---|---|---|
| Sequence | kobs/I (M-1s-1) | IC50 (nM) | Ki(nM) |
| NH-Idc-hGlu-Thr(Bzl)-Ser-Asp-AOMK | 665.000 | 7,9 | 0,7 |
| NH-Idc-hGlu-Thr(Bzl)-Lys-Asp-AOMK | 400.000 | 8,8 | 0,8 |
| NH-Idc-hGlu-Thr(Bzl)-Orn-Asp-AOMK | 390.000 | 7,4 | 0,7 |
| NH-Idc-hGlu-Thr(Bzl)-Dab-Asp-AOMK | 342.000 | 12,0 | 1,1 |
| NH-Idc-hGlu-Thr(Bzl)-Dap-Asp-AOMK | 372.000 | | |
| Ac-Val-Asp-Val-Ala-Asp-AOMK | 643.000 | 4,9 | 0,4 |

**Table 4**

| | Caspase-3 | | |
|---|---|---|---|
| Sequence | kobs/I (M-1s-1) | IC50 (nM) | Ki(nM) |
| NH-Idc-hGlu-Thr(Bzl)-Ser-Asp-AOMK | 21.000 | 7,1 | 1,1 |
| NH-Idc-hGlu-Thr(Bzl)-Lys-Asp-AOMK | 2.570 | 24,8 | 3,8 |
| NH-Idc-hGlu-Thr(Bzl)-Orn-Asp-AOMK | 636 | 91,0 | 13,8 |
| NH-Idc-hGlu-Thr(Bzl)-Dab-Asp-AOMK | 443 | 193,0 | 29,3 |
| NH-Idc-hGlu-Thr(Bzl)-Dap-Asp-AOMK | 8.300 | | |
| Ac-Val-Asp-Val-Ala-Asp-AOMK | 1.360.000 | 0,1 | 0,0 |

**Table 5**

| | Caspase-8 | | |
|---|---|---|---|
| Sequence | kobs/I (M-1s-1) | IC50 (nM) | Ki(nM) |
| NH-Idc-hGlu-Thr(Bzl)-Ser-Asp-AOMK | 11.600 | 46,2 | 6,2 |
| NH-Idc-hGlu-Thr(Bzl)-Lys-Asp-AOMK | 9.900 | 49,8 | 6,7 |
| NH-Idc-hGlu-Thr(Bzl)-Orn-Asp-AOMK | 2.300 | 320,0 | 43,3 |
| NH-Idc-hGlu-Thr(Bzl)-Dab-Asp-AOMK | 415 | 1540,0 | 209,0 |
| NH-Idc-hGlu-Thr(Bzl)-Dap-Asp-AOMK | 5.300 | | |
| Ac-Val-Asp-Val-Ala-Asp-AOMK | 98.400 | 16,0 | 2,2 |

Selectivity results are also presented as divisions of kobs/l, Ki and IC50 values (see tables 6 and 7 below).

**Table 6**

| | SELECTIVITY | | |
|---|---|---|---|
| | Caspase2 vs.Caspase3 | | |
| | **C2/C3** | **C3/C2** | **C3/C2** |
| Sequence | kobs/I (M-1s-1) | IC50 (nM) | Ki(nM) |
| NH-Idc-hGlu-Thr(Bzl)-Ser-Asp-AOMK | 31,7 | 0,9 | 1,5 |
| NH-Idc-hGlu-Thr(Bzl)-Lys-Asp-AOMK | 155,6 | 2,8 | 4,8 |
| NH-Idc-hGlu-Thr(Bzl)-Orn-Asp-AOMK | 613,2 | 12,4 | 20,3 |
| NH-Idc-hGlu-Thr(Bzl)-Dab-Asp-AOMK | 772,0 | 16,1 | 26,4 |
| NH-Idc-hGlu-Thr(Bzl)-Dap-Asp-AOMK | 44.8 | | |
| Ac-Val-Asp-Val-Ala-Asp-AOMK | 0,5 | 0,0 | 0,0 |

**Table 7**

| | SELECTIVITY | | |
|---|---|---|---|
| | Caspase2 vs.Caspase8 | | |
| | **C2/C8** | **C8/C2** | **C8/C2** |
| Sequence | kobs/I (M-1s-1) | IC50 (nM) | Ki(nM) |
| NH-Idc-hGlu-Thr(Bzl)-Ser-Asp-AOMK | 57,3 | 5,9 | 8,7 |
| NH-Idc-hGlu-Thr(Bzl)-Lys-Asp-AOMK | 40,4 | 5,7 | 8,4 |
| NH-Idc-hGlu-Thr(Bzl)-Orn-Asp-AOMK | 169,6 | 43,5 | 63,7 |
| NH-Idc-hGlu-Thr(Bzl)-Dab-Asp-AOMK | 824,1 | 128,3 | 188,3 |
| NH-Idc-hGlu-Thr(Bzl)-Dap-Asp-AOMK | 70.2 | | |
| Ac-Val-Asp-Val-Ala-Asp-AOMK | 6,5 | 3,3 | 4,9 |

As already indicated, compounds NH-ldc-hGlu-Thr(Bzl)-Ser-Asp-ACC and NH-ldc-hGlu-Thr(Bzl)-Ser-Asp-AOMK are disclosed as Caspase-2 substrate and inhibitor in Poreba et al., Cell Death & Differentiation 2019, 26, 2695-2709. As shown in the tables above, the inventors of the present invention have found that compounds of formula NH-ldc-hGlu-Thr(Bzl)-AA-Asp-AOMK with a Lys, Orn, Dab or Dap group in position AA, instead of, for example, Serine, present high Caspase-2 inhibitory activity and a remarkable selectivity over Caspase-3 or Caspase-8, thus leading to improved Caspase-2 inhibitors.

### Example 3. Intracellular lipid accumulation

### - Materials and methods

HepG2 cells (50.000 cells/well) were plated into 12-multiwell culture dishes on coverslips previously coated with collagen (2mg/mL collagen solution (BD Biosciences) in PBS incubated at 37°C for 30min).

Cells were treated with a "NASH cocktail": DMEM containing 4,5mg/mL glucose supplemented with 10% (v/v) FBS, 1% (v/v) PenStrep and 2mM L-glutamine plus fatty acids (100µM sodium oleate and 100µM palmitic acid), 100nM insulin (all Sigma-Aldrich) and inflammatory cytokines (50ng/mL tumor necrosis factor, TNF-α (Prospec), 25ng/mL interleukin IL-1β (Petroteck) and 8ng/mL transforming growth factor, TGF-β (R&D Systems)). The cells were exposed for 24h to this "NASH cocktail" and KIN compounds were concomitantly added at different concentrations.

At the end of treatments cells were washed with PBS and incubated with staining solution (BODIPY493/503 (Thermo Fisher Scientific) for 15 min at 37°C. Then cells were washed three times with PBS and were fixed in 4% PFA for 30 min at room temperature. After three additional washes with PBS, coverslips were mounted onto glass slides with a drop of Prolong^{®} Gold antifade reagent (Invitrogen) with DAPI overnight at room temperature. Fluorescence micrographs were taken using an Axoimagen M1 microscope (Zeiss, Oberkochen, Germany) and fluorescent signals (DAPI and BODIPY) were quantified using imaged (n=10). The intracellular lipid load was calculated as a function of BODIPYTM 493/503 lipid dye area.

### - Results

One metabolic process thought to initiate NASH is de novo lipogenesis (DNL), whose rate is up to 3-fold higher in patients (Lambert et al., 2014). DNL was speculated to contribute to NASH progression by increasing intracellular lipotoxic free fatty acids (FFA) in hepatocytes. Caspase-2 has been described to regulate the transcription of pro-lipogenic enzymes such as HMGCR (3-Hydroxy-3-Methylglutaryl-CoA Reductase) and HMGCS (hydroxymethylglutaryl-CoA synthase) (Kim, 2018). Caspase 2 inhibition could therefore prevent progression to NASH by diminishing the intracellular lipid concentration in hepatocytes.

An *in vitro* cellular model of NASH was used to test the efficacy of caspase-2 inhibitors preventing intracellular accumulation of lipids. In this model, HepG2 cells are exposed for 24 h to "NASH conditions" including lipogenic (glucose, insulin, fatty acids) and inflammatory and pro-apoptotic (TNF-α, IL- 1β, and TGF-β) triggers. Cells show a significantly increase in intracellular lipid load of at least 2-fold relative to the untriggered controls. This model has shown to correlate with human NASH pathology (Boeckmans et al. 2019).

NASH-triggered HepG2 cultures were treated with KIN inhibitors at 10 □M for 24 h and inhibition of intracellular lipid-accumulation was quantified as described in Material and Methods. As shown in Figure 4, all tested caspase-2 inhibitors were able to inhibit lipid accumulation under NASH-conditions. Compounds 1, 2 and 3 showed a significantly higher percentage of inhibition than NH-23-C2 compound.

The efficacy of Caspase-2 inhibitors in preventing intracellular lipid accumulation was used to compare the efficacy of NH-23-C2 and compound 1. Cells were incubated with NASH conditions and the caspase-2 inhibitors at 10, 20 and 25 □M to analyze its protection against lipid accumulation. As shown in Figure 5, compound 1 showed a better efficacy than NH-23-C2 inhibiting intracellular lipid accumulation under NASH conditions.

These experimental results provide experimental evidence that compounds of the invention, which are able to modulate the activity of Caspase-2 are efficient inhibiting lipid accumulation in hepatocytes.

### Example 4. kinetic assay

### - Materials and methods

Caspase-2 substrates were synthesized according to standard SPPS procedure, purified on HPLC, lyophilized and dissolved in DMSO to the final concentration of 20mM (Poreba et al. 2014, CDD). In particular, the following caspase-2 substrates were synthesized to carry out the present example:

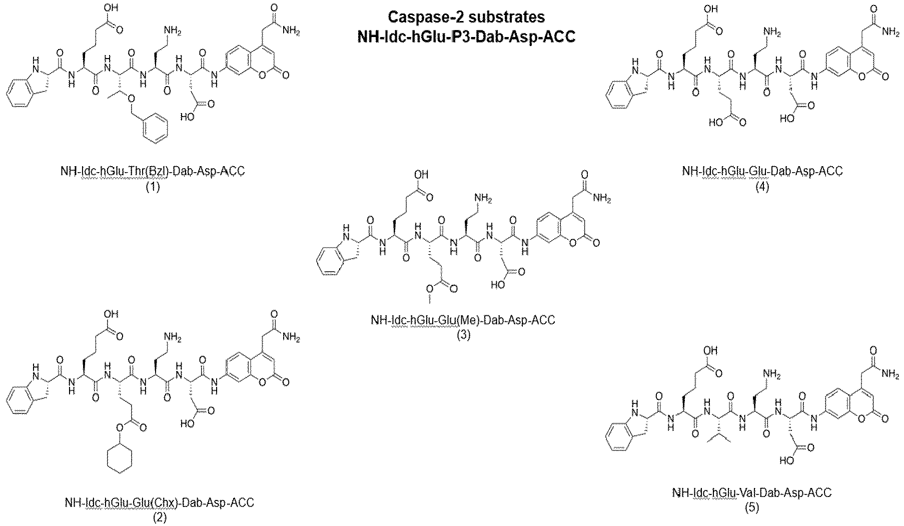

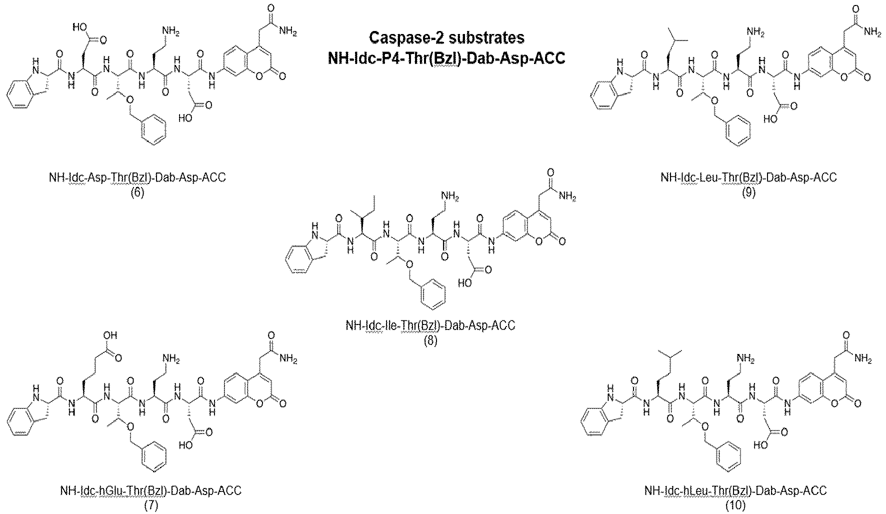

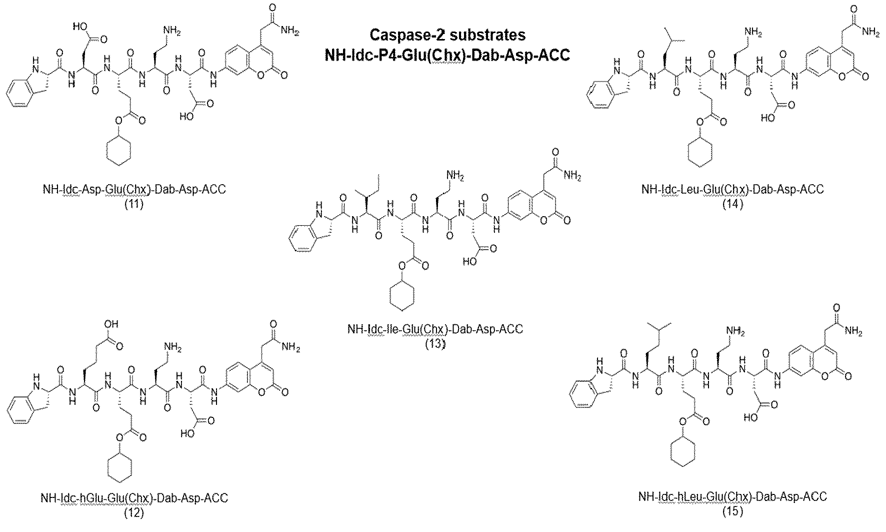

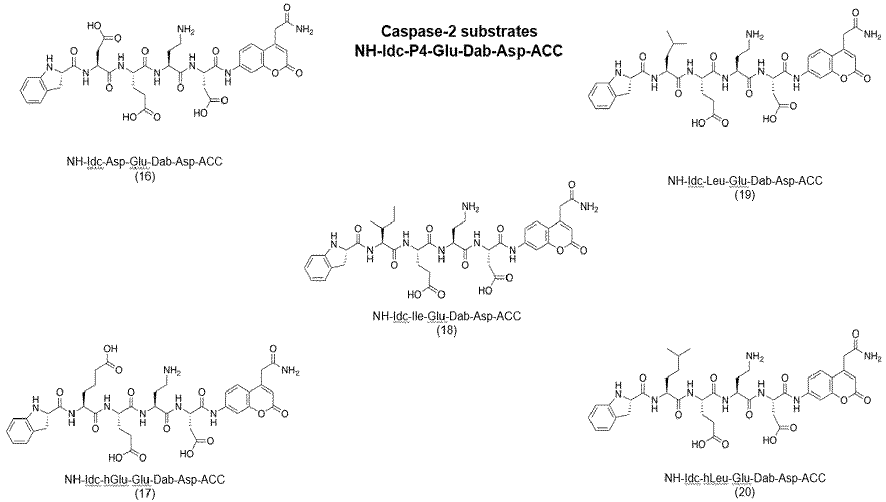

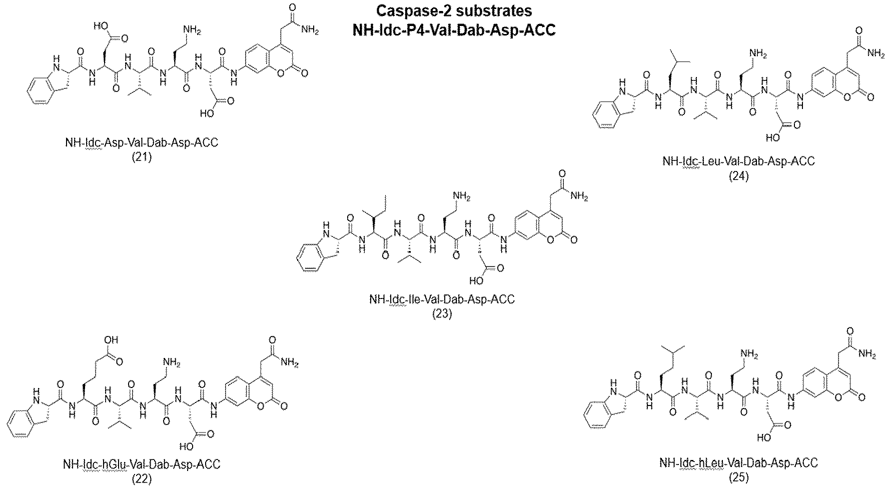

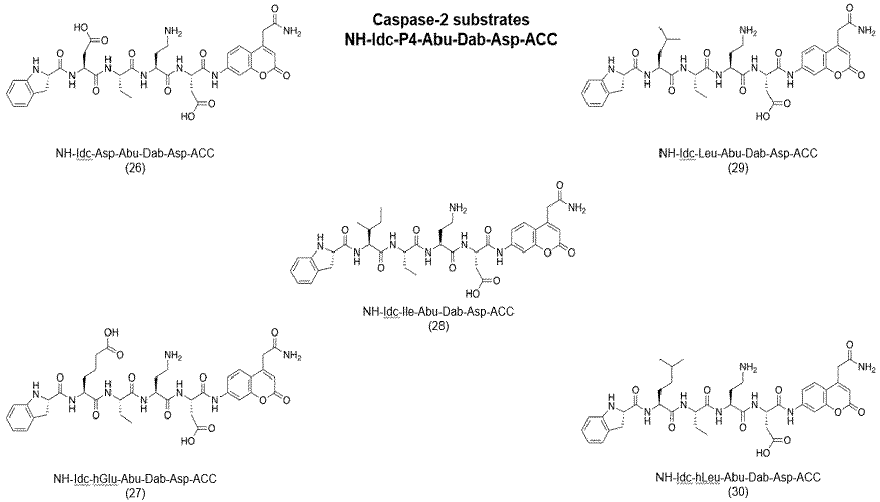

Prior to kinetic assay, substrates (several µL) were diluted in DMSO to the 1µM (working concentration). Next, 1µL of such substrate was spot into the well of 96-well plate following by the addition of 99 mL of caspase-2 in buffer. Immediately after the enzyme was added, the plate was placed in fluorescence plate reader and the fluorescence was measured over the time in a kinetic mode (excitation 355nm, emission 460nm). The liberation of fluorescence was carried out for 30 minutes, and only linear portion of the plot was taken for analysis to calculate the rate of the reaction. The rate of best cleaved substrate was set to 100% and all other substrates were adjusted accordingly (cleavage rate, %). The final concentration of substrate was 10µM, and the final concentration of caspase-2 was 10nM. The caspase-2 assay buffer was 20mM Pipes, 100mM NaCl, sucrose 10% (w/v), 10mM DTT, pH 7.2-7.4. The caspase-2 was preincubated in buffer at 37°C for 15 min before added to the substrate.

### Results

The results are summarized in figure 6 and indicated in detail in the following table:

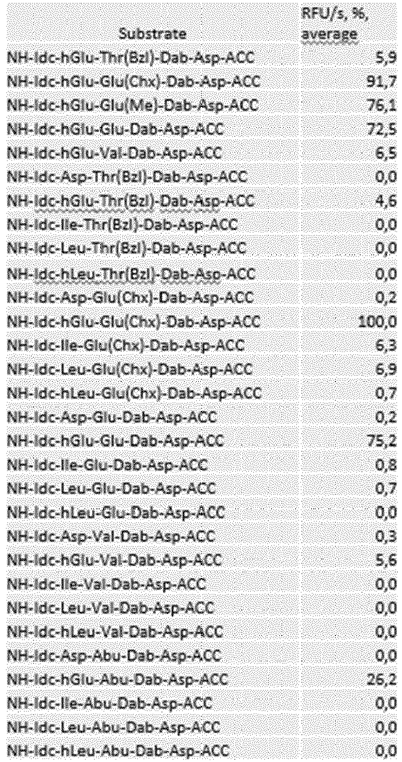

To assure data reliability all of the caspase 2 substrates were made independently (synthesis, purification, dilution in DMSO). The reference comparative value is the rate of hydrolysis of the best substrate from this particular series of substrates, namely NH-Idc-hGlu-Glu(Chx)-Dab-Asp-ACC. From this example it is clear that when a pentapeptide with the following scaffold NH-ldc-P4-P3-Dab-Asp-ACC is used, position P4 is much preferably hGlu and P3 can be left open to at least any of compounds Thr(Bzl), Glu(Chx), Glu(me), Glu or Val (also to Abu). This is further demonstrated in figure 7.

### Example 5.

### - Materials and methods

A series of caspase-2 substrates with the general formula NH-ldc-P4-Mix-Dab-Asp-ACC, where the P4 was either Asp, hGlu, Ile, Leu or hLeu; Mix was an equimolar mixture of natural amino acids, and ACC is a fluorescent tag, was synthesized on a solid support according to the general method described in Poreba et al, CDD, 2014. 500 mg (1 eq., 0.24 mmol) of Fmoc-protected Rink Amide resin (0.48 mol/g) was placed into glass cartridge for solid-phase synthesis and swollen in DCM for 30 min. Then the DCM was drained, and the resin was washed three times with DMF. Next, the Fmoc-protecting group was removed using 20% piperidine in DMF in three cycles (5 min, 5 min, and 25 min), and the resin was washed six times with DMF. About 2.5 eq. of Fmoc-ACC-OH (0.6 mmol, 265 mg) was pre-activated with 2.5 eq. of HATU (0.6 mmol, 228 mg) and 2.5 eq. of 2,4,6-collidine (0.6 mmol, 80 µL) in a minimal amount of DMF for 3 min and poured onto the resin. The reaction was carried out for 4 h, followed by filtration and washing the resin three times with DMF. After this time, the resin was washed three times with DMF and the ninhydrin test was performed to confirm the complete Fmoc-ACC-OH coupling. Then the Fmoc group was removed from ACC by using 20% piperidine in DMF, followed by washing the resin six times with DMF. Next, 2.5 eq. of Fmoc-L-Asp(tBu)-OH (0.6 mmol, 247 mg) was pre-incubated with 2.5 eq. of HATU (0.6 mmol, 228 mg) and 2.5 eq. of 2,4,6-collidine (0.6 mmol, 80µL) in DMF for 3 min and poured onto the H2N-ACC-resin. The reaction was carried out for 24 h and repeated for another 24 h with the use of 1.5 eq. of the Fmoc-L-Asp(tBu)-OH/HATU/2,4,6-collidine reagents. Then the Fmoc-L-Asp(tBu)-ACC-resin was washed three times with DMF, followed by Fmoc deprotection with 20% piperidine in DMF, and washing the resin six times with DMF. Next, Fmoc-L-Dab(Boc)-OH was coupled into P2 position. 2.5 eq. of this amino acid (0.6 mmol, 264 mg), 2.5 eq. of HATU (0.6 mmol, 229 mg), and 2.5 eq. of 2,4,6-collidine (0.6 mmol, 80 mL) was diluted into DMF and poured onto H2N-mix-Asp(tBu)-ACC-resin. The reaction was carried out for 3 h, followed by washing the slurry with DMF (six times), and performing a ninhydrin test to confirm complete coupling. The Fmoc group was removed with 20% piperidine in DMF, and the resin was washed six times with DMF. An isokinetic mixture of 19 natural amino acids (excluding cysteine, and with norleucine mimicking methionine) was coupled into P3 position. To do this, 5 eq. of the isokinetic mixture (1.2 mmol), 5 eq. of HOBt (1.2 mmol, 180 mg), and 5 eq. of DICI (1.2 mmol, 160 µL) were diluted in DMF and pre-activated for 3 min. Then the mixture was poured onto the resin and the cartridge was agitated for 3 h. The slurry was filtrated and washed three times with DMF. The P3 complete coupling was confirmed by a ninhydrin test. Next, the Fmoc group was removed with 20% piperidine in DMF and the H2N-Mix-Dab(Boc)-Asp(tBu)-ACC-resin was divided into 5 portion (0.05 mmol each). The P4 coupling was performed using a MultiChem 48-well synthesis apparatus (FlexChem from SciGene, CA, USA). 5 portions of resin were placed in the 48-well cartridge and swollen with DCM for 30 min. Then the DCM was filtrated, and resin was washed three times with DMF. In 5 1.5-mL tubes, 2.5 eq. of various amino acids (0.12 mmol) was mixed with 1 mL of DMF containing 2.5 eq. of HATU (0.12 mmol, 46 mg) and 2.5 eq. of 2,4,6-collidine (0.12 mmol, 16 µL) and poured onto the resin. The P4 coupling reaction was carried out for 4 h, followed by a ninhydrin test. Finally, the Fmoc group was removed with 20% piperidine in DMF, and the resin was washed with DMF 6 times. Next, Fmoc-L-ldc-OH was coupled into P5 position. 2.5 eq. of this amino acid (0.6 mmol, 231 mg), 2.5 eq. of HATU (0.6 mmol, 229 mg), and 2.5 eq. of 2,4,6-collidine (0.6 mmol, 80 mL) was diluted into DMF and poured onto each portion of H2N-P4-Mix-Dab(Boc)-Asp(tBu)-ACC-resin. The reaction was carried out for 3 h, followed by washing the slurry with DMF (six times), and performing a ninhydrin test to confirm complete coupling. The Fmoc group was removed with 20% piperidine in DMF, and the resin was washed six times with DMF, 3 times with DCM and 3 times with MeOH. Resin was dried out is a desiccator over P2O5 overnight. All substrates were cleaved from the resin using ice-cold TFA/TPS/water (% v/v/v, 95/2.5/2.5) mixture for 2 h (shaking once per 15 min). The solution from each well was collected separately and the remaining resin was washed with TFA. Substrates were then precipitated with ice-cold Et2O for 30 min and centrifuged. Next, the supernatant was discarded, and the pellet was re-suspended in ice-cold Et2O and centrifuged again. The supernatant was then discarded and the pellet was allowed to dry out, and was dissolved in 5 mL of 1:1 waterACN mixture, frozen at -80 °C and lyophilized. The final product (white powder) was then dissolved in dimethyl sulfoxide (DMSO) to the final concentration of 10 mM and stored at -80 °C until use.

1µL of substrate (10 mM) was spot into the well of 96-well plate following by the addition of 99mL of caspase-2 in buffer. Immediately after the enzyme was added, the plate was placed in fluorescence plate reader and the fluorescence was measured over the time in a kinetic mode (excitation 355nm, emission 460nm). The liberation of fluorescence was carried out for 30 min, and only linear portion of the plot was taken for analysis to calculate the rate of the reaction. The rate of best cleaved substrate was set to 100 % and all other substrates were adjusted accordingly (cleavage rate, %).

The composition of isokinetic mixture used in this particular synthesis (total amount - 1.2 mmol)

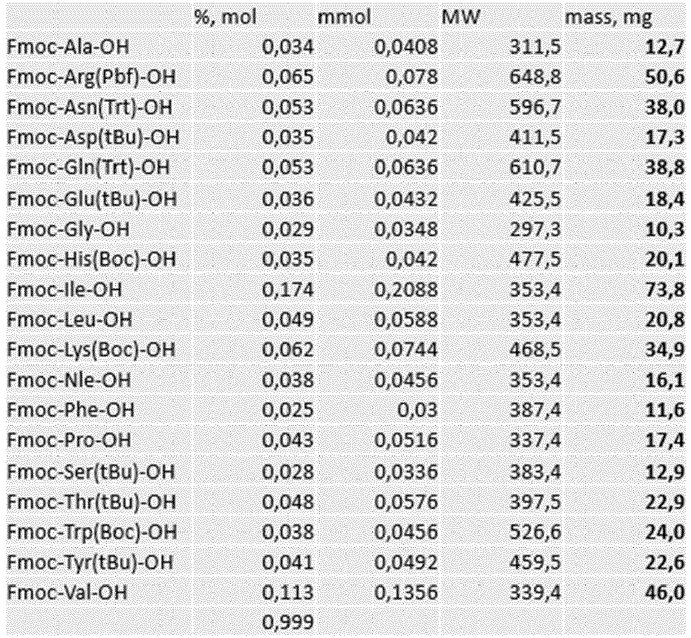

The final concentration of substrate was 10µM, and the final concentration of caspase-2 was 10nM. The caspase-2 assay buffer was 20mM Pipes, 100mM NaCl, sucrose 10% (w/v), 10mM DTT, pH 7.2-7.4. The caspase-2 was preincubated in buffer at 37 C degree for 15 min before added to the substrate.

The results are illustrated in figure 8 (left panel) and in the table below:

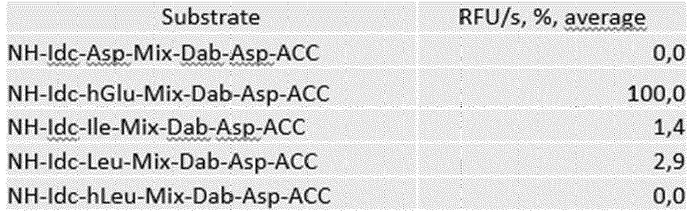

It is clear from the above table that P4 must be hGlu independently of the natural amino acid used in P3 which can be any of the natural amino acids selected from the list consisting of Ala (Alanine), Arg (Arginine), Asn (Asparagine), Asp (Aspartic Acid), Gln (Glutamine), Gly (Glycine), His (Histidine), lie (Isoleucine), Leu (Leucine), Lys (Lysine), Nle (Norleucine), Phe (Phenylalanine), Pro (Proline), Ser (Serine), Thr (Threonine), Trp (Tryptophan), Tyr (Tyrosine) and Val (Valine).

Finally, for a better representation of the rate of hydrolysis of the best substrates from each of the series shown in examples 4 and 5 was set to 100%, and other substrates were adjusted accordingly, the results are illustrated in figure 8.

### Example 6.

### Synthesis of Warhead building blocks

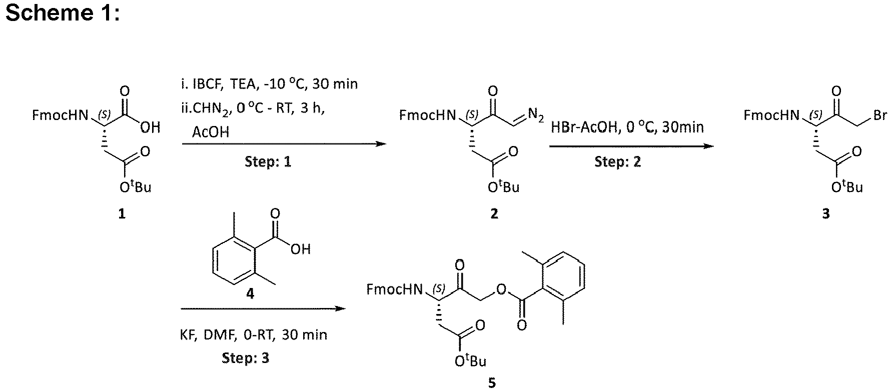

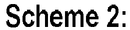

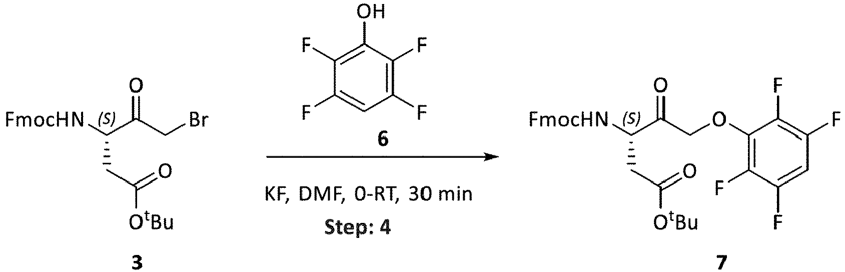

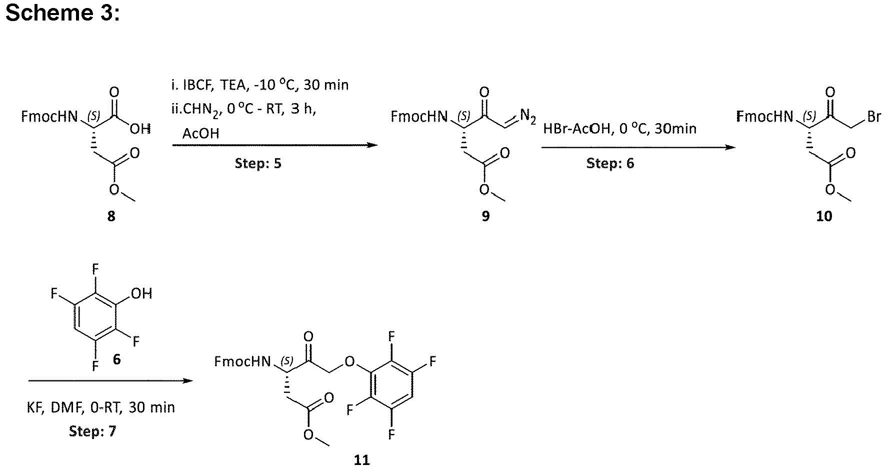

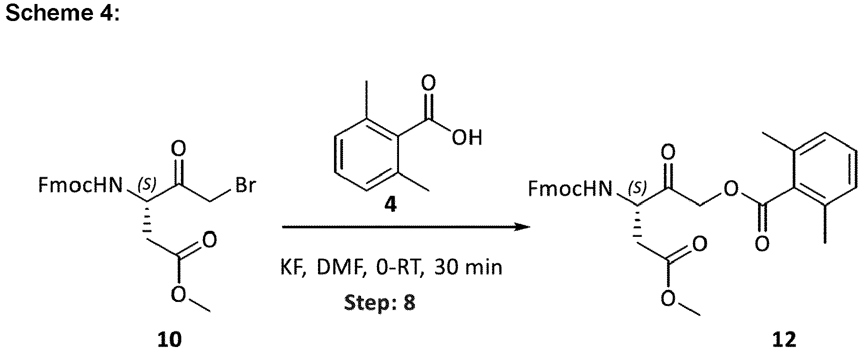

### Synthesis of Amino Acid building blocks:

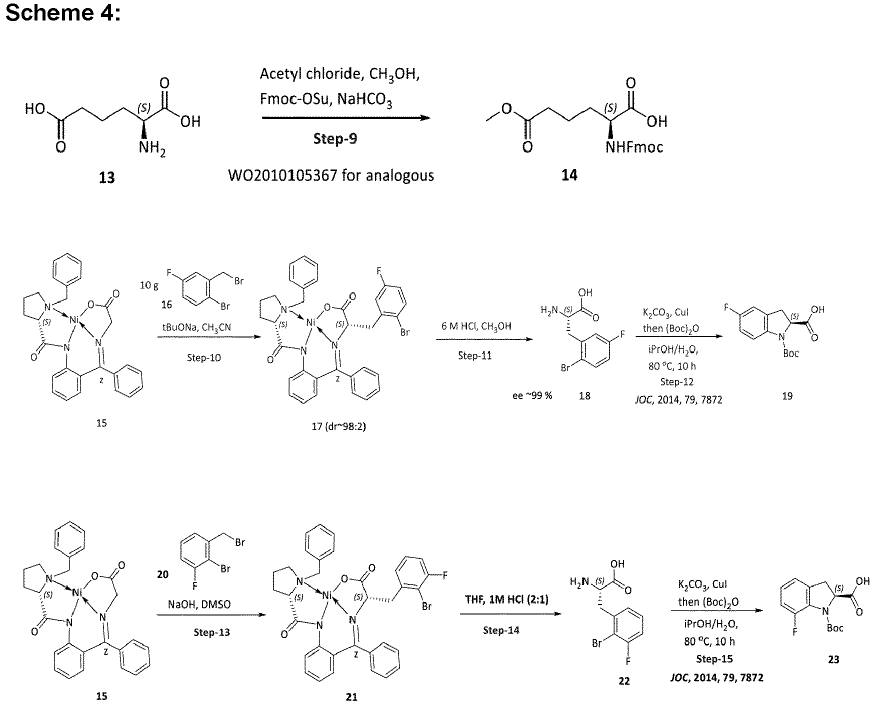

### General synthetic scheme for Caspase-2 candidates:

**Example-1: Cas 2_1: NH-ldc-hGlu-Thr(Bzl)-Dab-Asp-AOMK**
**Example-2: Cas 2_6: NH-ldc(7-F)-hGlu-Thr(Bzl)-Dab-Asp-AOMK**
**Example-3: Cas 2_9: NH-ldc(5-F)-hGlu-Thr(Bzl)-Dab-Asp-O(2,3,5,6-F4-Ph)**
**Example-4: Cas 2_9: NH-ldc(5-F)-hGlu-Thr(Bzl)-Dab-Asp-O(2,3,5,6-F4-Ph)**

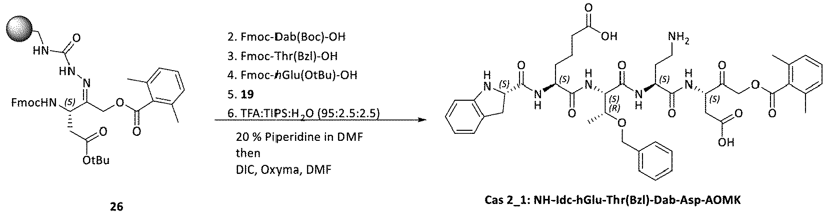

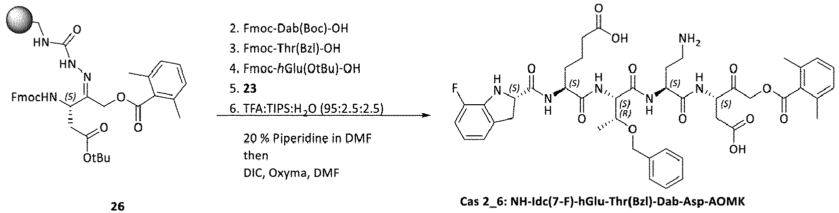

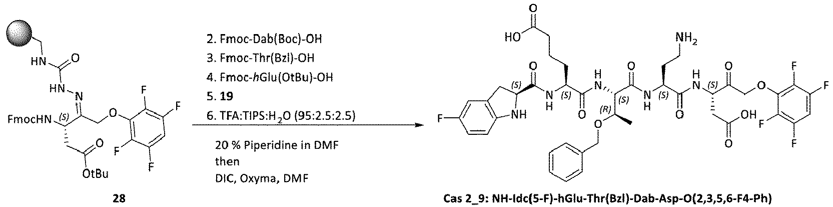

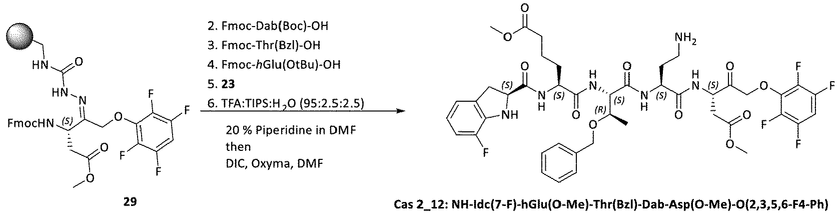

## Claims

1. A compound of formula (II): 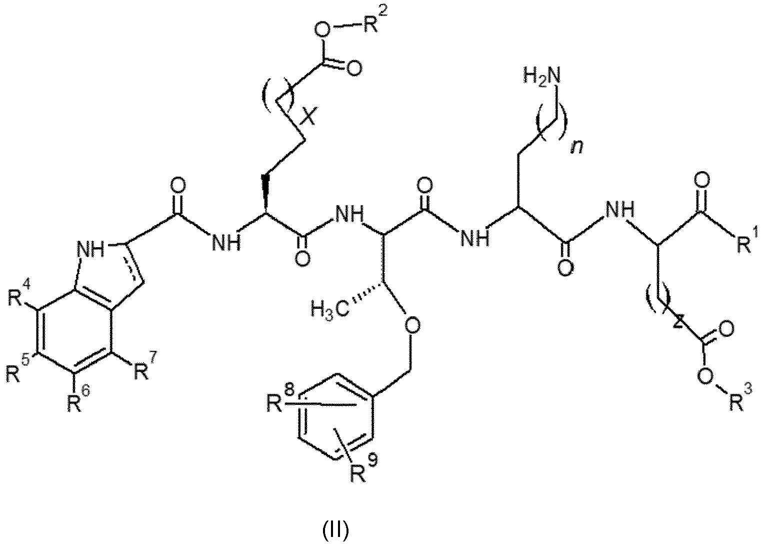
or a salt, solvate or stereoisomer thereof;
wherein: 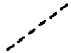
(the dotted line) in the heterocyclic ring of position P5 represents a bond which maybe present or absent, when present it forms a double bond in combination with the single bond already present;
R₂ and R₃ are independently selected from a hydrogen or a C₁₋₄ alkyl including branched alkyl and cycloalkyl;
R₄, R₅, R₆ and R₇ are each independently selected from a hydrogen, a halogen or a C₁₋₄ alkyl including branched alkyl and cycloalkyl, wherein each C₁₋₄ alkyl is optionally substituted with 1 -3 halogen atoms;
x and z are each independently selected from 0, 1, or 2; and n is 0, 1, 2 or 3;
R₈ and R₉ are independently selected from a hydrogen, a halogen or a C1-4 alkyl including branched alkyl and cycloalkyl, wherein each C1-4 alkyl is optionally substituted with 1 -3 halogen atoms; and
R₁ is a chemically reactive group such as a fluorophore or a warhead.

2. The compound of formula (II) of claim 1, wherein 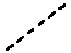 (the dotted line) in the heterocyclic ring of position P5 represents a bond which maybe present or absent, when present it forms a double bond in combination with the single bond already present;
R₄, R₅, R₆ and R₇ are each independently selected from a hydrogen, a halogen such as a fluorine or a trifluoromethyl;
x, and z are each 1;
n is 0, 1, 2 or 3;
R₈ and R₉ are each independently selected from a hydrogen, a halogen such as a fluorine or a trifluoromethyl;
R₂ and R₃ are independently selected from a hydrogen or a C₁₋₄ alkyl, preferably methyl or ethyl; and
R₁ is a chemically reactive group as defined in claim 1.

3. The compound of formula (II) of claim 1,
wherein: 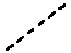 (the dotted line) in the heterocyclic ring of position P5 represents a bond which maybe present or absent, when present it forms a double bond in combination with the single bond already present;
R₄, R₅, R₆ and R₇ are each independently selected from a hydrogen or a halogen such as a fluorine;
x, and z are each 1;
n is 0, 1, 2 or 3;
R₈ and R₉ are each independently selected from a hydrogen, a halogen such as a fluorine or a trifluoromethyl;
R₂ and R₃ are independently selected from a hydrogen or a methyl; and
R₁ is a chemically reactive group as defined in claim 1.

4. The compound of formula (II) of claim 1,
wherein: 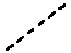 (the dotted line) in the heterocyclic ring of position P5 represents a bond which maybe present or absent, when present it forms a double bond in combination with the single bond already present;
R₄, and R₆ are each independently selected from a hydrogen or a halogen such as a fluorine and R₅ and R₇ are both hydrogens;
x, and z are each 1;
n is 0, 1, 2 or 3;
R₈ and R₉ are each independently selected from hydrogen, a halogen such as a fluorine or a trifluoromethyl;
R₂ and R₃ are independently selected from hydrogen or methyl; and
R₁ is a chemically reactive group as defined in claim 1.

5. The compound of formula (II) of claim 1,
wherein: 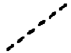 (the dotted line) in the heterocyclic ring of position P5 represents a bond which maybe present or absent, when present it forms a double bond in combination with the single bond already present;
R₄, and R₆ are each independently selected from a hydrogen, or a halogen such as a fluorine and R₅ and R₇ are both hydrogens;
x, and z are each 1;
n is 0, 1, 2 or 3;
R₈ and R₉ are both hydrogens;
R₂ and R₃ are independently selected from hydrogen or methyl; and
R₁ is a chemically reactive group as defined in claim 1.

6. The compound of claim 1, wherein said compound has general formula (III), or a salt, solvate or stereoisomer thereof, 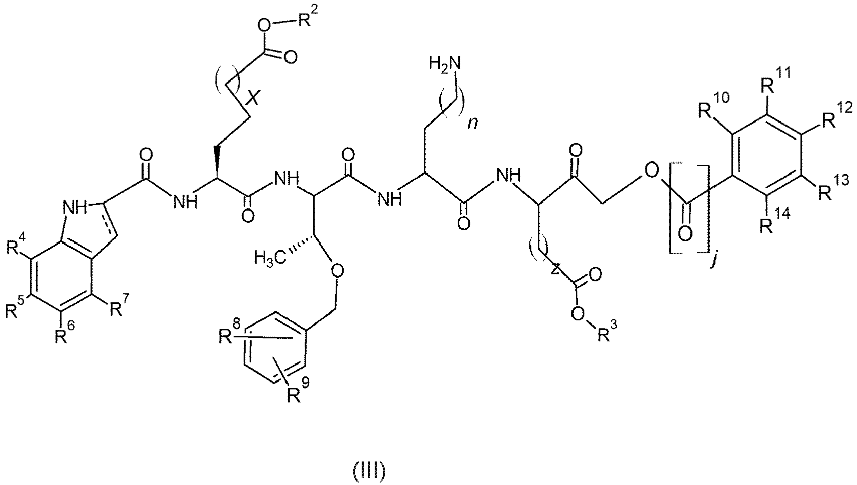 wherein 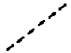
(the dotted line) in the heterocyclic ring of position P5 represents a bond which maybe present or absent, when present it forms a double bond in combination with the single bond already present;
R₂ and R₃ are independently selected from a hydrogen or a C₁₋₄ alkyl including branched alkyl and cycloalkyl;
R₄, R₅, R₆ and R₇ are each independently selected from a hydrogen, a halogen or a C₁₋₄ alkyl including branched alkyl and cycloalkyl, wherein each C₁₋₄ alkyl is optionally substituted with 1 -3 halogen atoms;
x and z are each independently selected from 0, 1, or 2, and n is 0, 1, 2 or 3;
R₈ and R₉ are independently selected from a hydrogen, a halogen or a C1-4 alkyl including branched alkyl and cycloalkyl, wherein each C1-4 alkyl is optionally substituted with 1 -3 halogen atoms;
j is 0 or 1; and
R₁₀, R₁₁, R₁₂, R₁₃ and R₁₄ are each independently selected from hydrogen, halogen or a C1-4 alkyl including branched alkyl and cycloalkyl, phenyl, phenoxy, nitro, nitrile, PhCO, NHAc, wherein each alkyl is optionally substituted with 1 -3 halogen atoms.

7. The compound of claim 6,
wherein: 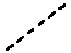
(the dotted line) in the heterocyclic ring of position P5 represents a bond which maybe present or absent, when present it forms a double bond in combination with the single bond already present;
R₄, R₅, R₆ and R₇ are each independently selected from a hydrogen, a halogen such as a fluorine or a trifluoromethyl;
x, and z are each 1;
n is 0, 1, 2 or 3;
R₈ and R₉ are each independently selected from a hydrogen, a halogen such as a fluorine or a trifluoromethyl;
R₂ and R₃ are independently selected from a hydrogen or a C₁₋₄ alkyl, preferably methyl or ethyl;
j is 0 or 1; and
R₁₀, R₁₁, R₁₂, R₁₃ and R₁₄ are each independently selected from a hydrogen, halogen or a C1-4 alkyl including branched alkyl and cycloalkyl, phenyl, phenoxy, nitro, nitrile, PhCO, NHAc, wherein each alkyl is optionally substituted with 1 -3 halogen atoms.

8. The compound of claim 1, wherein said compound has general formula (IV), or a salt, solvate or stereoisomer thereof, 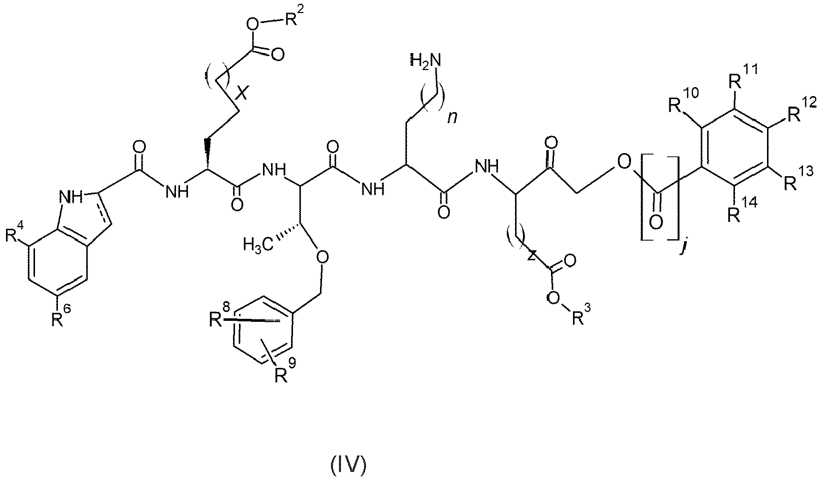 wherein 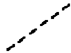
(the dotted line) in the heterocyclic ring of position P5 represents a bond which maybe present or absent, when present it forms a double bond in combination with the single bond already present;
R₂ and R₃ are independently selected from a hydrogen, or a C₁₋₄ alkyl including branched alkyl and cycloalkyl, preferably R₂ and R₃ are independently selected from a hydrogen, a methyl or an ethyl;
R₄, and R₆ are each independently selected from a hydrogen, a halogen or a C₁₋₄alkyl including branched alkyl and cycloalkyl, wherein each C₁₋₄ alkyl is optionally substituted with 1 -3 halogen atoms; preferably, R₄, and R₆ are each independently chosen from a hydrogen, a halogen such as a fluorine or a trifluoromethyl;
x and z are each independently selected from 0, 1, or 2 and n is 0, 1, 2 or 3; preferably x, and z are 1 and n is 0, 1, 2 or 3;
R₈ and R₉ are independently selected from a hydrogen, a halogen or a C₁₋₄ alkyl including branched alkyl and cycloalkyl, wherein each C₁₋₄ alkyl is optionally substituted with 1 -3 halogen atoms; preferably, R₈ and R₉ are independently selected from a hydrogen or a halogen such as fluorine;
j is 0 or 1; and
R₁₀, R₁₁, R₁₂, R₁₃ and R₁₄ are each independently selected from a hydrogen, a halogen or a C1-4 alkyl including branched alkyl and cycloalkyl, phenyl, phenoxy, nitro, nitrile, PhCO, NHAc, wherein each alkyl is optionally substituted with 1 -3 halogen atoms; preferably, R₁₄ or R₁₀ are selected from a halogen such as fluorine, a methyl or a trifluoromethyl, and R₁₁, R₁₂, and R₁₃ are each a hydrogen or a halogen such as fluorine.

9. The compound of claim 1, wherein said compound has general formula (V), or a salt, solvate or stereoisomer thereof, 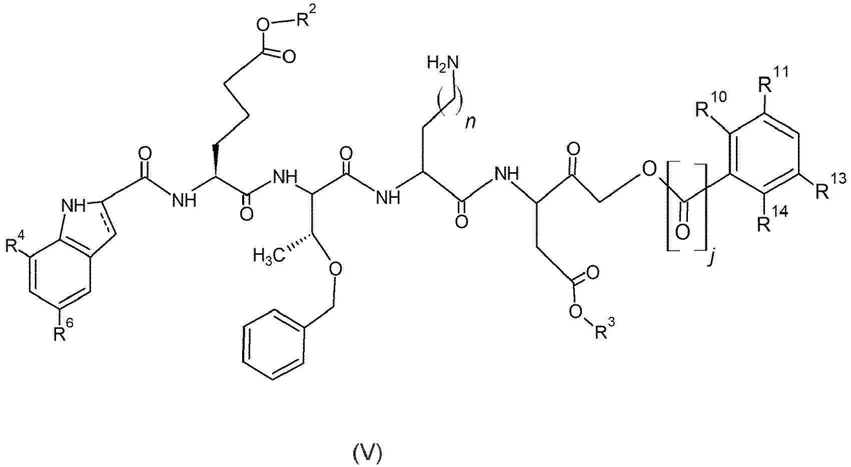 wherein 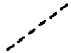
(the dotted line) in the heterocyclic ring of position P5 represents a bond which maybe present or absent, when present it forms a double bond in combination with the single bond already present;
R₂ and R₃ are independently selected from a hydrogen or a methyl;
R₄, and R₆ are each independently selected from a hydrogen, or a halogen such as a fluorine;
n is 0, 1, 2 or 3;
j is 0 or 1; and
R₁₄ or R₁₀ are selected from a halogen such as fluorine, or a methyl and R₁₁, and R₁₃ are each a hydrogen or a halogen such as fluorine;
whereas when j is 0, then R₁₄, R₁₀ are a halogen such as fluorine, and R₁₁, and R₁₃ are each a halogen such as fluorine.

10. The compound of claim 1, wherein said compound is selected from any one of the following chemical structures (protonated or deprotonated) or a salt, solvate or stereoisomer thereof:
| | |
|---|---|
| 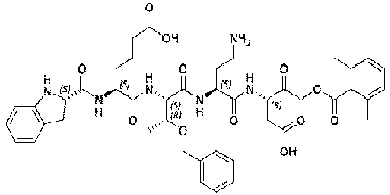 | 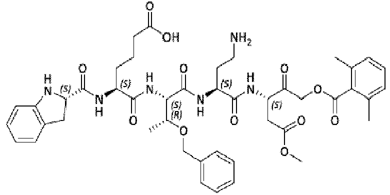 |
| **Cas 2_1: NH-Idc-hGlu-Thr(Bzl)-Dab-Asp-AOMK** | **Cas 2_2: NH-Idc-hGlu-Thr(Bzl)-Dab-Asp(O-Me)-AOMK** |
| 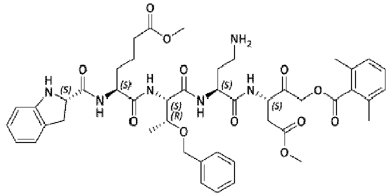 | 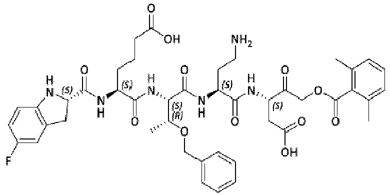 |
| **Cas 2_3: NH-Idc-hGlu(O-Me)-Thr(Bzl)-Dab-Asp(O-Me)-AOMK** | **Cas 2_4: NH-Idc(5-F)-hGlu-Thr(Bzl)-Dab-Asp-AOMK** |
| 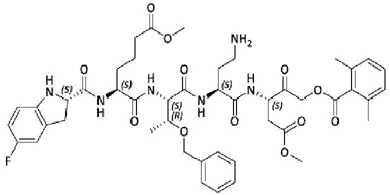 | 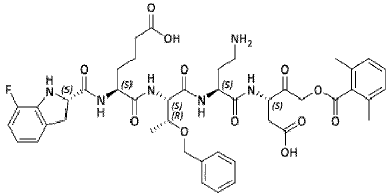 |
| **Cas 2_5: NH-Idc(5-F)-hGlu(O-Me)-Thr(Bzl)-Dab-Asp(O-Me)-AOMK** | **Cas 2_6: NH-Idc(7-F)-hGlu-Thr(Bzl)-Dab-Asp-AOMK** |
| 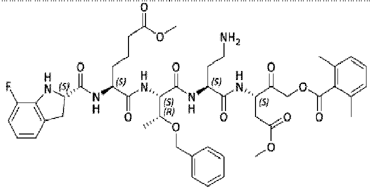 | 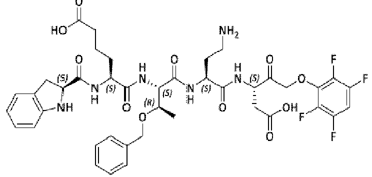 |
| **Cas 2_7: NH-Idc(7-F)-hGlu(O-Me)-Thr(Bzl)-Dab-Asp(O-Me)-AOMK** | **Cas 2_8: NH-Ide-hGlu-Thr(Bzl)-Dab-Asp-O(2,3,5,6-F4-Ph)** |
| 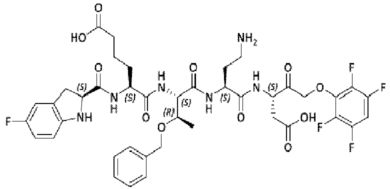 | 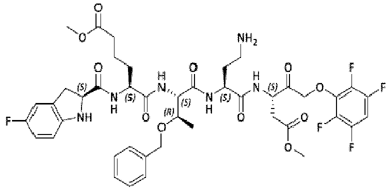 |
| **Cas 2_9: NH-Idc(5-F)-hGlu-Thr(Bzl)-Dab-Asp-O(2,3,5,6-F4-Ph)** | **Cas 2_10: NH-Idc(5-F)-hGlu(O-Me)-Thr(Bzl)-Dab-Asp(O-Me)-O(2,3,5,6-F4-Ph)** |
| 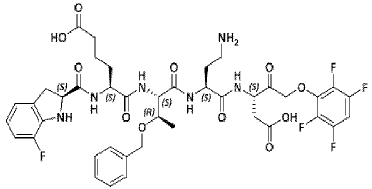 | 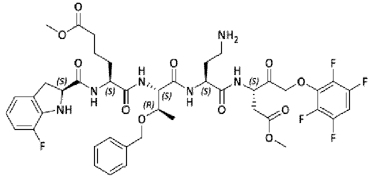 |
| **Cas 2_11**: **NH-Idc(7-F)-hGlu-Thr(Bzl)-Dab-Asp-O(2,3,5,6-F4-Ph)** | **Cas 2_12: NH-Idc(7-F)-hGlu(O-Me)-Thr(Bzl)-Dab-Asp(O-Mo]-O(2,3,5,6-F4-Ph)** |

11. Compound according to any of the precedent claims, wherein all the amino acids in the compound are in the L configuration.

12. Pharmaceutical composition comprising a compound according to any one of claims 1 to 10 and optionally a pharmaceutically acceptable excipient.

13. Compound according to any one of claims 1 to 10, for use as a medicament.

14. Compound according to any one of claims 1 to 10, for use in the prevention and/or treatment of degenerative diseases such as Alzheimer's disease, Huntington's disease, Parkinson's disease, Lewy body dementia, mild cognitive impairment, amyotrophic lateral sclerosis and Creutzfeld-Jacob disease; neonatal brain damage in particular neonatal brain ischemia; traumatic brain injury; kidney ischemia; hypoxia-ischemia injuries; stroke-like situations brain injuries; heart ischemia; myocardial infarction; amyotrophic lateral sclerosis; retinal damages; ophthalmic diseases such as blunt ocular injury, ischemic optic neuropathy and glaucoma; skin damages; sterile inflammatory diseases such as diabetes, atherosclerosis, cardiac ischemia, gout, pseudogout, joint loosening, atherosclerosis, syndromes triggered by aluminium salts, non-arteritic ischemic optic neuropathy, glaucoma and metabolic diseases; non-sterile inflammatory diseases such as bacterial infection in particular with bacteria producing pore-forming toxins, influenza virus infection and single-stranded RNA Rhabdoviridae infection, such as Maraba virus or vesicular stomatitis virus; diseases caused by pathogenic bacteria, such as Brucella, Staphylococcus aureus and Salmonella; dyslipidemias; obesity; metabolic syndrome; and nonalcoholic fatty liver disease, such as nonalcoholic steatohepatitis (NASH) and nonalcoholic fatty liver disease (NAFLD), cirrhosis, primary sclerosing cholangitis and hepatocellular carcinoma.

15. Compound according to any one of claims 1 to 10, for use in the prevention and/or treatment of dyslipidemias, obesity, metabolic syndrome, cirrhosis, nonalcoholic steatohepatitis (NASH) or nonalcoholic fatty liver disease (NAFLD).
